(19)

Europäisches
Patentamt

European
Patent Office

Office européen
des brevets

(11)  **EP 3 173 483 A1**

(12)  **EUROPEAN PATENT APPLICATION**

(43) Date of publication:
**31.05.2017  Bulletin 2017/22**

(51) Int Cl.:
*C12N 15/113* (2010.01)    *C07K 16/22* (2006.01)
*C07K 16/24* (2006.01)     *A61K 39/00* (2006.01)
*A61K 31/7088* (2006.01)   *A61P 35/00* (2006.01)

(21) Application number: **15382590.6**

(22) Date of filing: **27.11.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicants:
• **Fundació Privada Institut d'Investigació
Oncològica de Vall-Hebron
08035 Barcelona (ES)**
• **Institució Catalana de Recerca i Estudis Avançats
08010 Barcelona (ES)**

(72) Inventors:
• **SEOANE SUAREZ, Joan
08035 Barcelona (ES)**

• **SOTO SIMÓN, Atenea
08035 Barcelona (ES)**
• **SALA HOJMAN, Ada
08035 Barcelona (ES)**
• **HUBER RUANO, Isabel
08035 Barcelona (ES)**
• **CHIGANCAS, Vanesa
08035 Barcelona (ES)**
• **ANIDO FOLGUEIRA, Judit
08035 Barcelona (ES)**

(74) Representative: **ABG Patentes, S.L.
Avenida de Burgos, 16D
Edificio Euromor
28036 Madrid (ES)**

(54)  **AGENTS FOR THE TREATMENT OF DISEASES ASSOCIATED WITH UNDESIRED CELL PROLIFERATION**

(57)  The present invention relates to methods for the treatment of from ovarian, lung or colorectal cancer by means of the use of agents capable of inhibiting the expression and/or capable of blocking the activity of leukemia inhibitory factor (LIF). The invention also relates to *in vitro* methods for designing a customised therapy for a patient suffering from a ovarian, lung or colorectal cancer and for selecting a patient suffering from a ovarian, lung or colorectal cancer to be treated with an agent capable of inhibiting the expression and/or capable of blocking the activity of LIF.

**EP 3 173 483 A1**

**Description**

**TECHNICAL FIELD**

The present invention relates to the field of therapy and, more in particular, to the field of cancer therapeutics by using inhibitors of LIF.

**BACKGROUND ART**

Cancer is now primarily treated with one or a combination of three types of therapies: surgery; radiation; and chemotherapy. Surgery involves the bulk removal of diseased tissue. While surgery is sometimes effective in removing tumors located at certain sites, for example, in the breast, colon, and skin, it cannot be used in the treatment of tumors located in other areas, such as the backbone, nor in the treatment of disseminated neoplastic conditions such as leukemia. Radiation therapy involves the exposure of living tissue to ionizing radiation causing death or damage to the exposed cells. Side effects from radiation therapy may be acute and temporary, while others may be irreversible. Chemotherapy involves the disruption of cell replication or cell metabolism. It is used most often in the treatment of breast, lung, and testicular cancer. The adverse effects of systemic chemotherapy used in the treatment of neoplastic disease are most feared by patients undergoing treatment for cancer. Of these adverse effects, nausea and vomiting are the most common. Other adverse side effects include cytopenia, infection, cachexia, mucositis in patients receiving high doses of chemotherapy with bone marrow rescue or radiation therapy; alopecia (hair loss); cutaneous complications such as pruritis, urticaria, and angioedema; neurological complications; pulmonary and cardiac complications; and reproductive and endocrine complications. Chemotherapy-induced side effects significantly impact the quality of life of the patient and may dramatically influence patient compliance with treatment. As such, improved methods of treatment are needed.

LIF is an Interleukin 6 (IL-6)-type cytokine that is involved in a variety of biological activities and has effects on different cell types. Human LIF is a polypeptide of 202 amino acids. It has been described that LIF is involved in the activation of the JAK-STAT cascade mediated by TGFβ, thus inducing the cell proliferation process and the increase of tumor stem cells (cancer stem cells) in glioma. Based on this fact, it has been shown that LIF inhibitors are useful for the treatment of glioma showing high LIF expression levels, being said activity mediated by the capacity of LIF of inhibiting proliferation of glioma inhibiting cells.

**BRIEF SUMMARY OF THE INVENTION**

The authors of the present invention have found that inhibitors of LIF are able to efficiently reduce progression of ovarian, lung and colorectal cancer. In particular, the authors of the present invention have observed that anti-LIF neutralizing antibodies are capable of reducing tumor growth of NSCLC in an orthotopic model in a manner that depends on the expression levels of LIF in the tumor. Similarly, anti-LIF neutralizing antibodies have been shown to inhibit tumor growth in ortothopic models of ovarian cancer and in colorectal cancer.

Thus, in a first aspect, the present invention is related to an agent capable of inhibiting the expression and/or capable of blocking the activity of leukemia inhibitory factor (LIF) for use in the treatment of a cancer selected from ovarian cancer, lung cancer and colorectal cancer.

In another aspect, the invention relates to an *in vitro* method for designing a customised therapy for a patient suffering from a cancer selected from ovarian cancer, lung cancer and colorectal cancer, comprising:

(i) quantifying the levels of LIF in a sample from said patient, and
(ii) comparing said levels with a reference value,

wherein if the levels of LIF in said sample from said patient are equal or greater than the reference value, then an agent capable of inhibiting the expression and/or capable of blocking the activity of LIF is selected for administration to said patient.

In yet another aspect, the invention relates to an *in vitro* method for selecting a patient suffering from a cancer selected from ovarian cancer, lung cancer and colorectal cancer to be treated with an agent capable of inhibiting the expression and/or capable of blocking the activity of LIF, comprising:

(i) quantifying the levels of LIF in a sample from said patient, and
(ii) comparing said levels with a reference value,
wherein if the levels LIF in said sample from said patient are equal or greater than the reference value, then said patient is selected to receive treatment with an agent capable of inhibiting the expression and/or capable of blocking the activity of LIF.

## BRIEF DESCRIPTION OF THE FIGURES

**Figure 1:** LIF expression in different cancer cell types.
**Figure 2:** Heterogeneity of LIF expression in glioblastoma, NSCLC, ovarian and pancreatic tumors.
**Figure 3:** Determination of the LIF score.
**Figure 4:** The inhibitory effect on tumor growth of the anti-LIF antibody depends on the levels of LIF in the tumor.
**Figure 5:** Anti-LIF antibody promotes NSCLC tumor regression *in vivo.*
**Figure 6:** Anti-LIF antibody therapeutic effect in NSCLC tumors depends on LIF levels.
**Figure 7:** the LIF pathway is critical for NSCLC tumor growth.
**Figure 8:** Anti-LIF antibody inhibits tumor growth in a glioblastoma xenograft model (U251).
**Figure 9:** Tumor growth in glioblastoma depends of LIF levels.
**Figure 10:** Anti-LIF antibody inhibits tumor growth in an ovarian orthotopic immunocompetent model (ID8).
**Figure 11:** Ovarian tumor growth depends on LIF level in the ID8 model.
**Figure 12:** Anti-LIF antibody inhibits tumor growth in a colorectal cancer immunocompetent model (CT26).

## DETAILED DESCRIPTION OF THE INVENTION

*Definitions*

The expression "agent blocking activity", or "agent capable of blocking activity", as used herein, relates to any inhibitory substance or compound that is capable of preventing the protein encoded by a gene from performing its function (activity), i.e., preventing LIF from being able to induce the activation of the JAK-STAT signalling pathway. Exemplary inhibitory agents capable of preventing the protein encoded by the gene coding LIF from performing its function are, for example, inhibitory peptides of the protein, antibodies directed specifically against epitopes of the protein essential for carrying out its function and which are capable of neutralizing the activity of LIF, i.e. blocking signaling by the receptor in response to binding to LIF, or against LIF receptors, etc.

The expression "agent inhibiting the expression" or "inhibitor of the expression", or "agent capable of inhibiting the expression", as used herein, relates to any inhibitory substance or compound that is capable of preventing or blocking the transcription and/or the translation of a gene, particularly of a gene encoding LIF, i.e. capable of preventing or blocking the expression of said gene. By way of illustration, inhibitory agents of the expression of LIF suitable according to the present invention are, for example, antisense oligonucleotides, interference RNAs (siRNAs), catalytic RNAs or specific ribozymes, RNA with decoy activity, i.e., with capacity to bind specifically to a factor (generally proteinaceous) important for the expression of the gene, etc.

The terms "antibody", "immunoglobulin" and the like terms refer to a polypeptide substantially encoded by an immunoglobulin gene or immunoglobulin genes, or fragments thereof, which specifically bind and recognize an analyte (antigen). The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. An exemplary immunoglobulin (antibody) structural unit is composed of two pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains respectively. The C-terminal ends of each heavy chain are disulfide bonded together, and form the constant region of the antibody. Depending on the amino acid sequence of the constant domain of their heavy chains, antibodies can be assigned to different "classes". There are five-major classes of antibodies: IgA, IgD, IgE, IgG, and IgM, and several of these may be further divided into "subclasses" (isotypes), e.g., IgG1, IgG2, IgG3, IgG4, IgA, and IgA2. Full-length immunoglobulin "light chains" (of about 25 kDa or about 214 amino acids) comprise a variable region of about 1-10 amino acids at the NH2-terminus and a kappa or lambda constant region at the COOH-terminus. Full-length immunoglobulin "heavy chains" (of about 50 kDa or about 446 amino acids) similarly comprise a variable region (of about 116 amino acids) and one of the aforementioned heavy chain constant regions or classes, e.g., gamma (of about 330 amino acids). The subunit structures and three-dimensional configurations of different classes of immunoglobulins are well known.

The term "cancer" or "tumor" or "tumor disease" or "neoplasm", as used herein, refers to a broad group of diseases involving unregulated cell growth and which are also referred to as malignant neoplasms. The term is usually applied to a disease characterized by uncontrolled cell division (or by an increase of survival or apoptosis resistance) and by the ability of said cells to invade other neighboring tissues (invasion) and spread to other areas of the body where the cells are not normally located (metastasis) through the lymphatic and blood vessels, circulate through the bloodstream, and then invade normal tissues elsewhere in the body. Cancers usually share some of the following characteristics: sustaining proliferative signalling, evading growth suppressors, resisting cell death, enabling replicative immortality, inducing angiogenesis, and activating invasion and eventually metastasis. Cancers invade nearby parts of the body and may also spread to more distant parts of the body through the lymphatic system or bloodstream. Cancers are classified by the type of cell that the tumor cells resemble, which is therefore presumed to be the origin of the tumor. The term cancer includes, without limitation, lung cancer, sarcoma, malignant melanoma, pleural mesothelioma, bladder carcinoma, prostate cancer, pancreas carcinoma, gastric carcinoma, ovarian cancer, hepatoma, breast cancer, colorectal cancer, kidney cancer, esophageal cancer, suprarenal cancer, parotid gland cancer, head and neck carcinoma, cervix cancer, endometrial cancer, liver cancer, mesothelioma, multiple myeloma, leukemia, and lymphoma.

In a particular embodiment of the invention, the cancer is selected from the group consisting of colorectal cancer, lung cancer and ovarian cancer. The term "colorectal cancer", also known as "colon cancer", "rectal cancer", or "bowel cancer", refers to a cancer from uncontrolled cell growth in the colon or rectum, or in the appendix. The term is used to refer to adenocarcinomas, carcinoid tumors, Gastrointestinal stromal tumors (GISTs) or sarcomas. As used herein, the term colorectal cancer refers to stage I, stage IIA, stage IIB, stage IIC, stage IIIA, stage IIIB, stage IIIC, stage IVA or stage IVB colorectal cancer Moreover, as used herein, colorectal cancer refers both to primary colorectal tumors as well as to secondary colorectal cancer, i.e. a colorectal cancer which results from the metastasis from a primary cancer elsewhere in the body.

The term "lung cancer" relates to the cancer arising from the cells of the respiratory epithelium, and can be divided into two broad categories. The term lung cancer, as used herein, refers to small cell lung cancer (SCLC) or non-small cell lung cancer (NSCLC), including adenocarcinoma, squamous cell carcinoma, and large cell carcinoma. In a particular embodiment, the lung cancer is NSCLC. As used herein, the term lung cancer refers to stage IA, stage IB, stage IIA, stage IIB, stage IIIA stage IIIB or stage IV lung cancer. Moreover, as used herein, lung cancer refers both to primary lung tumors as well as to secondary lung cancer, i.e. lung cancer which results from the metastasis from a primary cancer elsewhere in the body.

The term "ovarian cancer" relates to a cancerous growth arising from the ovary. As used herein, ovarian cancer is used to refer to both type I cancers (endometrioid, mucinous, and clear-cell carcinomas) as well as to type II cancers (serous carcinoma and carcinosarcoma). As used herein, ovarian cancer refers to surface epithelial-stromal tumor (adenocarcinoma), papillary serous cystadenocarcinoma, "Borderline" adenocarcinoma, adenocarcinoma, endometrioid tumor, serous cystadenocarcinoma, papillary carcinoma, mucinous cystadenocarcinoma, clear-cell ovarian tumor, mucinous adenocarcinoma, cystadenocarcinoma, sex cord-stromal tumour, Mullerian tumor, germ cell tumor, teratoma, dysgerminoma, epidermoid (squamous cell carcinoma) or Brenner tumor. As used herein, ovarian cancer refers to stage I, stage II, stage III or stage IV ovarian cancer. Moreover, as used herein, ovarian cancer refers both to primary ovarian tumors as well as to secondary ovarian cancer, i.e. an ovarian cancer which results from the metastasis from a primary cancer elsewhere in the body.

The term "H-score", as used herein, relates to a parameter which defines the expression levels of a given protein in by staining using immunohistochemistry. In the immunohistochemistry, each tumor cell is given an intensity level ranging from 0 for no staining to 3+ for the most intense staining, being 2 for the moderately staining cells and 1 for the weakly staining cells. The H-score is then determined using the following formula:

$$\text{H-score} = 3 \times \text{percentage of strongly staining cells} + 2 \times \text{percentage of moderately staining cells} + 1 \times \text{percentage of weakly staining cells}$$

Accordingly, the value of the H-score can range from 0 wherein no cells shows any staining to 300, wherein 100% cells in the sample show strong staining.

The term "leukemia inhibitory factor", also known as "LIF", relates to an interleukin 6 class cytokine that affects cell growth by inhibiting differentiation. It is capable of inducing the terminal differentiation of myeloid leukemic cells, thus preventing their continued growth. Human LIF protein sequence is located at UniProt database under accession number P15018 (version as of 16 May 2014). Human gene encoding LIF protein is located at NBCI database under Gene ID:3976 (version as of 4 October 2015). In a preferred embodiment, LIF refers to the 202 amino acids human isoform 1, corresponding to the polypeptide having accession number P15018-1 in the UniProt database (entry of November 11, 2015). In another embodiment, LIF refers to the 88 amino acids human LIF isoform 2. In another embodiment, LIF refers to the combination of human LIF isoforms 1 and 2.

The term "LIF score", as used herein, relates to a parameter used to quantify LIF expression levels in a test sample. The LIF-score in a sample is determined by staining the sample with the anti-LIF specific antibody using immunohistochemistry. In the immunohistochemistry, each tumor cell is given an intensity level ranging from 0 for no staining to 3+ for the most intense staining, being 2 for the moderately staining cells and 1 for the weakly staining cells. The LIF-score is then determined as the percentage (from 0% to 100%) of cells with a strong complete staining (3+) for LIF. Accordingly, the LIF-score can have values ranging from 0 (when no cell in the sample has a 3+ staining) to 100 (when 100% cells of the sample have a 3+ staining). The LIF score in a sample can be used directly to provide an indication as to LIF expression levels or can be compared to a reference LIF score value to provide an indication as to whether LIF expression levels are increased, decreased or show no variation with respect to the reference value.

The term "reference value", as used herein, relates to a predetermined criteria used as a reference for evaluating the values or data obtained from the samples collected from a subject. The reference value or reference level can be an absolute value; a relative value; a value that has an upper or a lower limit; a range of values; an average value; a median value; a mean value; or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

The term "sample" or "biological sample", as used herein, refers to biological material isolated from a subject. The biological sample contains any biological material suitable for detecting RNA or protein levels. In a particular embodiment, the sample comprises genetic material, e.g., DNA, genomic DNA (gDNA), complementary DNA (cDNA), RNA, heterogeneous nuclear RNA (hnRNA), mRNA, etc., from the subject under study. The sample can be isolated from any suitable tissue or biological fluid such as, for example blood, saliva, plasma, serum, urine, cerebrospinal liquid (CSF), feces, a surgical specimen, a specimen obtained from a tumor biopsy or from a non-tumor biopsy, and a tissue sample embedded in paraffin. In one embodiment, the sample is a sample containing tumor cells. Methods for isolating samples are well known to those skilled in the art. In particular, methods for obtaining a sample from a biopsy include gross apportioning of a mass, or micro-dissection or other art-known cell-separation methods. In order to simplify conservation and handling of the samples, these can be formalin-fixed and paraffin-embedded or first frozen and then embedded in a cryosolidifiable medium, such as OCT-Compound, through immersion in a highly cryogenic medium that allows rapid freeze. In a particular embodiment of the invention, the sample is a tissue sample or a biofluid. In a more particular embodiment of the invention, the biofluid is selected from the group consisting of blood, serum or plasma. In a particular embodiment of the invention, the sample is a tumor sample.

The term "subject", or "individual" or "animal" or "patient" includes any subject, particularly a mammalian subject, for whom therapy is desired. Mammalian subjects include humans, domestic animals, farm animals, and zoo or pet animals such as dogs, cats, guinea pigs, rabbits, rats, mice, horses, cattle, cows, and so on. In a preferred embodiment of the invention, the subject is a mammal. In a more preferred embodiment of the invention, the subject is a human.

The term "treatment", as used herein, comprises any type of therapy, which aims at terminating, preventing, ameliorating and/or reducing the susceptibility to a clinical condition as described herein, e.g. cancer. In a preferred embodiment, the term treatment relates to prophylactic treatment (i.e. a therapy to reduce the susceptibility of a clinical condition, a disorder or condition as defined herein). Thus, "treatment," "treating," and the like, as used herein, refer to obtaining a desired pharmacologic and/or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or at least symptoms associated therewith, so that the host no longer suffers from the disorder or its symptoms, such as causing regression of the disorder or its symptoms, for example, by restoring or repairing a lost, missing or defective function, or stimulating an inefficient process, or (4) relieving, alleviating, or ameliorating the disorder, or symptoms associated therewith, where ameliorating is used in a broad sense to refer to at least a reduction in the magnitude of a parameter.

## *Agents inhabiting LIF expression or blocking LIF activity for use in the treatment of cancer*

The inventors of the present invention have found that LIF inhibitors are useful in the treatment of ovarian cancer, lung cancer and colorectal cancer.

Therefore, in a first aspect, the present invention relates to an agent capable of inhibiting the expression and/or capable of blocking the activity of LIF for use in the treatment of a cancer selected from ovarian cancer, lung cancer and colorectal cancer. Alternatively, the invention relates to a method for the treatment of cancer, wherein said cancer is selected from ovarian cancer, lung cancer and colorectal cancer, that comprises administering to a subject in need thereof a therapeutically effective amount of an agent capable of inhibiting the expression and/or capable of blocking the activity of LIF. Alternatively, the invention relates to the use of an agent capable of inhibiting the expression and/or capable of blocking the activity of LIF in the manufacture of a medicament for the treatment of cancer, wherein said cancer is selected from ovarian cancer, lung cancer and colorectal cancer.

In another aspect, the invention relates to an agent capable of inhibiting the expression and/or capable of blocking the activity of LIF for use in preventing the relapse of cancer selected from ovarian cancer, lung cancer and colorectal cancer. In a preferred embodiment, relapse is prevented in patients wherein the tumor has been surgically excised.

In another aspect, the invention relates to an agent capable of inhibiting the expression and/or capable of blocking the activity of LIF for use in preventing the metastasis of cancer selected from ovarian cancer, lung cancer and colorectal cancer.

In another aspect, the invention relates to an agent capable of inhibiting the expression and/or capable of blocking the activity of LIF for use in preventing the proliferation of cancer stem cells or for reducing the pool of cancer stem cells in a cancer selected from ovarian cancer, lung cancer and colorectal cancer.

In another aspect, the invention relates to an agent capable of inhibiting the expression and/or capable of blocking the activity of LIF for use in preventing the proliferation of cancer stem cells or for reducing the pool of cancer stem cells in a cancer selected from ovarian cancer, lung cancer and colorectal cancer, for use in preventing the relapse of cancer selected from ovarian cancer, lung cancer and colorectal cancer or for use in preventing the metastasis of cancer selected from ovarian cancer, lung cancer and colorectal cancer wherein the agent acts by inhibiting activation of the JAK/STAT pathway and phosphorylation of STAT3.

Agents capable of inhibiting the expression and/or capable of blocking the activity of LIF include any substance or compound capable of preventing or blocking the transcription and the translation of a gene encoding LIF, or that is capable of preventing the protein encoded by said gene from performing its function (activity), that is to say, preventing LIF from being able to induce the activation of the JAK-STAT signaling pathway.

Assays for determining if a compound is an inhibitory agent of LIF are well known in the state of the art. These assays include, without limitation, the measurement of STAT-3 phosphorylation by an IL-6 type cytokine in neurospheres and/or or tumor cells (WO2010/115868).

By way of illustration, inhibitory agents of the expression of LIF suitable for their use in the present invention include, for example, antisense oligonucleotides, interference RNAs (siRNAs), catalytic RNAs or specific ribozymes, RNA with decoy activity, i.e., with capacity to bind specifically to a factor (generally proteinaceous) important for the expression of the gene, etc. Likewise, inhibitory agents capable of preventing the LIF protein from performing its function include, for example, inhibitory peptides of the protein, antibodies directed specifically against epitopes of the protein essential for carrying out its function, or against LIF receptors, etc.

Therefore, in a particular embodiment of the invention, the LIF inhibitory agent for use according to the invention is selected from the group consisting of siRNAs, antisense oligonucleotides, specific ribozymes, antibodies, polypeptides and inhibitors of LIF.

In a particular embodiment, the agent for use according to the invention capable of inhibiting the expression of LIF is selected from the group consisting of a siRNA specific for LIF, an antisense oligonucleotide specific for LIF and a ribozyme specific for LIF.

In a particular embodiment, the agent for use according to the invention capable of blocking the activity of LIF is selected from the group consisting of an antibody specific for LIF, a polypeptide specific for LIF, an oligonucleotide specific for LIF and an inhibitor of LIF receptor binding specific for LIF. More particularly, the agent for use according to the invention capable of blocking the activity of LIF is an antibody, preferably a LIF neutralizing antibody.

*siRNA*

Small interfering RNAs, or siRNAs, are agents which are capable of inhibiting the expression of a target gene by means of RNA interference. A siRNA can be chemically synthesized, can be obtained by means of *in vitro* transcription or can be synthesized *in vivo* in the target cell. A siRNA typically consists of double-stranded RNA of between 15 and 40 nucleotides in length and it can contain a 3' and/or 5' overhang region of 1 to 6 nucleotides. The length of the overhang region is independent of the total length of the siRNA molecule. siRNAs act by means of degradation or posttranscriptional silencing of the target messenger.

siRNAs include shRNA (short hairpin RNA) characterized in that the antiparallel strands forming the siRNA are connected by a loop or hairpin region. These siRNAs are compounds of a short antisense sequence (from 19 to 25 nucleotides) followed by a loop of between 5 and 9 nucleotides which is followed by the sense strand. shRNAs can be encoded by plasmids or viruses, particularly retroviruses and more particularly retroviruses and can be under the control of promoters such as the U6 promoter of the RNA polymerase III. The siRNAs of the invention are substantially homologous to the mRNA of the gene encoding LIF or to the genomic sequence encoding said protein. "Substantially homologous" is understood as having a sequence which is sufficiently complementary or similar to the target mRNA such that the siRNA is capable of causing the degradation of the latter by RNA interference. The siRNAs suitable for causing said interference include siRNA formed by RNA, as well as siRNA containing different chemical modifications such as:

- siRNA in which the bonds between the nucleotides are different from those found naturally such as phosphorothioate bonds.
- conjugates of the RNA strand with a functional reagent, such as a fluorophore.
- modifications of the ends of the RNA strands, particularly the 3' end by means of the modification with different functional groups of the hydroxyl at position 2'
- nucleotides with modified sugars such as O-alkylated residues at position 2' such as 2'-O-methylribose p 2'-O-fluororibose.
- nucleotides with modified bases such as halogenated bases (for example 5-bromouracil and 5-iodouracil), alkylated bases (for example 7-methylguanosine).

The region of the nucleotide sequence which is taken as a basis for designing siRNA is not limiting and can contain a region of the encoding sequence (between the start codon and the end codon), or it can alternatively contain sequences of the 5' or 3' non-translated region, preferably between 25 and 50 nucleotides in length and in any position at position 3' with respect to the start codon. One way of designing an siRNA involves the identification of the AA(N19)TT motifs in which N can be any nucleotide in the sequence encoding an IL-6 type cytokine and selecting those which have a high G/C content. If said motif is not found, it is possible to identify the NA(N21) motif in which N can be any nucleotide.

Exemplary, non-limitative, siRNAs for LIF that are commercially available include human LIF siRNA from Santa Cruz Biotechnology (reference sc-37222), and human LIF siRNA from Thermo Fisher Scientific (reference AM16708).

*Antisense oligonucleotides*

An additional aspect of the invention relates to the use of isolated "antisense" nucleic acids to inhibit expression, for example inhibiting the transcription and/or translation of a nucleic acid encoding LIF, the activity of which is to be inhibited.

An antisense construct of the present invention can be delivered, for example, as an expression plasmid which, when transcribed in the cell, produces RNA complementary to at least a single part of the cell mRNA encoding LIF. Alternatively, the antisense construct is an oligonucleotide probe, which is generated *ex vivo* and which, when introduced in the cell, produces inhibition of the expression by hybridizing with the mRNA and/or genomic sequences of a target nucleic acid. Such oligonucleotide probes are preferably modified oligonucleotides which are resistant to endogenous nucleases, for example, exonucleases and/or endonucleases, and which are therefore stable in vivo. Exemplary nucleic acid molecules for their use as antisense oligonucleotides are phosphoramidate, phospothionate and methylphosphonate DNA analogs. General approaches for constructing oligomers useful in antisense therapy are well known in the art.

With respect to antisense DNA, the regions of oligodeoxyribonucleotides derived from the translation initiation site, for example, between -10 and +10 of the target gene, are preferred. Antisense approaches involve the design of oligonucleotides (either DNA or RNA) which are complementary to the mRNA encoding the target polypeptide. Antisense oligonucleotides will bind to the mRNA transcripts and prevent translation. Absolute complementarity is not required, though it is preferred. In the case of double stranded antisense nucleic acids, a single strand of the double-stranded DNAs can thus be assayed, or the formation of triple-stranded DNAs can be assayed. The capacity of hybridizing will depend both on the degree of complementarity and on the length of the antisense nucleic acid. Generally, the longer the nucleic acid hybridizing, the more RNA pairing errors it may contain and it still forms a stable duplex (or triplex, as the case may be). The person skilled in the art can determine a tolerable degree of pairing errors by means of the use of standard processes for determining the melting point of the hybridized complex. Oligonucleotides which are complementary to the 5' end of the mRNA, for example the non-translated 5' sequence up to and including the AUG start codon, must function as effectively as possible in order to inhibit translation. However, it has recently been shown that the sequences complementary to the non-translated 3' sequences of mRNA are also effective for inhibiting the translation of mRNAs. Therefore, oligonucleotides complementary to either the non-translated, non-encoding 5' or 3' regions of a gene could be used in an antisense approach to inhibit the translation of that mRNA. Oligonucleotides complementary to the non-translated 5' region of the mRNA should include the complement of the AUG start codon. Oligonucleotides complementary to the encoding regions of mRNA are less effective inhibitors of translation but they could also be used according to the invention. If designed to hybridize with the 5', 3', or encoding region of mRNA, antisense nucleic acids should have at least six nucleotides in length, and preferably have less than about 100 and more preferably less than about 50, 25, 17 or 10 nucleotides in length.

Antisense oligonucleotides can be of DNA or RNA or chimeric mixtures or modified derivatives or versions thereof, single-stranded or double-stranded. Oligonucleotide can be modified in the base group, the sugar group, or the phosphate backbone, for example to improve the stability of the molecule, hybridization etc. The oligonucleotide can include other bound groups such as peptides (for example, to direct them towards host cell receptors), or agents to make transport, through the cell membrane easier or the blood-brain barrier, hybridization triggered cleaving agents, intercalating agents. For this purpose, the oligonucleotide can be conjugated to another molecule, for example, a peptide, a hybridization triggered cross-linking agent, a carrier agent, a hybridization triggered cleaving agent, etc.

Antisense oligonucleotides can comprise at least one modified base group which is selected from the group including, but not limited to, 5-fluorouracil, 5-bromouracil, 5-chlorouracil, 5-iodouracil, hypoxanthine, xanthine, 4-acetylcytosine, 5-(carboxyhydroxytiethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1- methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methyl ester, uracil-5-oxyacetic acid (v), 5-methyl-2-thiouracil, 3-(3-amino-3-N-2-carboxypropyl) uracil, (acp3)w, and 2,6-diaminopurine.

The antisense oligonucleotide can also comprise at least one modified sugar group selected from the group including but not limited to arabinose, 2-fluoroarabinose, xylulose, and hexose. The antisense oligonucleotide can also contain a backbone similar to neutral peptide. Such molecules are referred to as peptide nucleic acid (PNA) oligomers. An advantage of PNA oligomers is their capacity to bind to complementary DNA in a manner essentially independent of the ion force of the medium due to the neutral backbone of DNA. In yet another embodiment, the antisense oligonucleotide comprises at least one modified phosphate backbone selected from the group consisting of a phosphorothioate, a phosphorodithioate, a phosphoramidothioate, a phosphoramidate, a phosphorodiamidate, a methylphosphonate, an alkyl phosphotriester, and a formacetal or analog thereof.

In yet another embodiment, the antisense oligonucleotide is an alpha-anomeric oligonucleotide. An alpha-anomeric oligonucleotide forms specific double-stranded hybrids with complementary RNA in which, in contrast with the typical antiparallel orientation, the strands are parallel to one another. The oligonucleotide is a 2'-O-methylribonucleotide or an RNA-DNA chimeric analog.

While antisense oligonucleotides complementary to the encoding region of the target mRNA sequence can be used, those complementary to the non-translated transcribed region can also be used.

In some cases, it may be difficult to reach intracellular concentrations of the antisense sufficient for suppressing the translation of the endogenous mRNAs. Therefore, a preferred approach uses a recombinant DNA construct in which the antisense oligonucleotide is placed under the control of a strong pol III or pol II promoter. The use of such construct for transfecting target cells will result in the transcription of sufficient amounts of single stranded RNAs which will form complementary base pairs with the potential target endogenous transcripts of drugs and will therefore prevent translation. For example, a vector can be introduced such that it is captured by a cell and directs the transcription of an antisense RNA. Such vector can remain episomal or be integrated in the chromosome, while it can be transcribed to produce the desired antisense RNA. Such vectors can be constructed by means of methods of recombinant DNA technology standard in the art. The vectors can be viral plasmids, or other plasmids known in the art used for replication and expression in mammal cells. The expression of the sequences encoding the antisense RNA can be by means of any promoter known in the art which acts on mammal cells, preferably human cells. Such promoters can be inducible or constitutive. Such promoters include but are not limited to: promoter of the SV40 early region, the promoter contained at 3' long repetition terminal of the Rous sarcoma virus, the herpes thymidine kinase promoter, the metallothionein gene regulatory sequences, etc. Any type of plasmid, cosmid, YAC or viral vector can be used to prepare the recombinant DNA construct, which can be introduced directly in the site of the tissue.

The expression of the target gene can alternatively be inhibited by directing complementary deoxyribonucleotide sequences to the regulatory region of the gene (i.e., the promoter and/or enhancers) to form triple helix structures preventing the transcription of the gene in the target cells in the body.

The potential target sequences which can be selected for the formation of triple helices can alternatively be increased, creating a nucleic acid molecule called "hairpin shaped". The hairpin-shaped molecules are synthesized in an alternating 5'-3', 3'-5' form, such that they forma first base pair with a strand of a duplex and then with the other one, eliminating the need for the presence of a rather large section of purines or pyrimidines in a strand of a duplex.

In some embodiments, the antisense oligonucleotides are antisense morpholines. Morpholines are synthetic molecules that are the product of a redesign of natural nucleic acid structure. Typically 25 bases in length, they bind to complementary RNA sequences by means of standard nucleic acid base pairing.

Examples of LIF-specific antisense oligonucleotides are described in Kamohara et al. (Int J Oncol, 2007, 30:977-983) and Cheng et al. (Biol Reprod, 2004 1 70:1270-1276).

*DNA enzymes*

Another aspect of the invention relates to the use of DNA enzymes to inhibit the expression of the gene encoding LIF. The DNA enzymes incorporate some of the mechanistic characteristics of both antisense and ribozymes technologies. The DNA enzymes are designed such that they recognize a particular target nucleic acid sequence, similar to the antisense oligonucleotide, however like ribozymes, they are catalytic and specifically cleave the target nucleic acid.

There are currently two types of DNA enzymes, and both were identified by Santoro and Joyce (see, for example, US patent 6,110,462). DNA enzyme 10-23 comprises a loop structure connecting two arms. The two arms provide specificity by recognizing a particular target nucleic acid sequence while the loop structure provides the catalytic function in physiological conditions.

*Ribozymes*

Ribozyme molecules designed to catalytically cleave transcripts of a target mRNA can also be used to prevent the translation of the mRNAs encoding LIF, the activity of which is to be inhibited. Ribozymes are enzymatic RNA molecules capable of catalyzing specific RNA cleaving. The composition of the ribozyme molecules preferably includes one or more sequences complementary to the target mRNA, and the well-known sequence responsible for cleaving the mRNA or a functionally equivalent sequence (see US patent 5,093,246, for example, herein incorporated by reference in its entirety).

While ribozymes cleaving mRNA into site-specific recognition sequences can be used to destroy target mRNAs, the use of hammerhead ribozymes is preferred. Hammerhead ribozymes cleave the mRNA at locations commanded by the flanking regions forming complementary base pairs with those of the target mRNA. Preferably, the target mRNA has the following two base sequence: 5'-UG-3'. The sequences of the hammerhead ribozyme can be embedded in stable RNA, such as transfer RNA (tRNA) to increase the efficacy of the *in vivo* cleavage. Particularly the expression of fusion ribozymes with tRNA mediated by RNA polymerase III is well known in the art. There is typically a number of potential cleavage sites of hammerhead ribozymes in a target cDNA sequence. The ribozyme is preferably manipulated such that the cleavage recognition site is located close to the 5' end of the target mRNA to increase the efficacy and minimize intracellular accumulation of non-functional mRNA transcripts. Furthermore, the use of any cleavage recognition site located in the target sequence encoding different parts of e-terminal amino acid domains of, for example, short and long forms of the target, would allow selectively directing towards either form of the target, and thus having a selective effect on a form of the target gene product.

Ribozymes directed against genes necessarily contain a hybridization region complementary to two regions, each of at least 5 and preferably each of 6, 7, 8, 9, 10, 11, 12, 13, 14, 15, 16 1 17, 18, 19 or 20 contiguous nucleotides in length of a target mRNA, such as an mRNA of a sequence represented in any of the human RAPBO proteins. Furthermore, ribozymes have very specific endonuclease activity which autocatalytically cleaves the encoding target mRNA. The present invention extends to ribozymes hybridizing with an encoding mRNA encoding a target gene such as a candidate target gene of a therapeutic drug, therefore hybridizing with the encoding mRNA and cleaving it, such that it is no longer capable of being translated for synthesizing a functional polypeptide product.

The ribozymes used in the compositions of the present invention also include endoribonuclease RNA (hereinafter "Cech-type ribozymes") such as that found naturally in *Tetrahymena thermophila* (known as IVS, or L-19 IVS RNA). The ribozymes can be formed by modified oligonucleotides (for example to improve stability, guidance, etc.) and they should be delivered to cells expressing the target gene *in vivo.* A preferred method for delivery involves using a DNA construct "encoding" the ribozyme under the control of a strong constitutive promoter of pol III or pol II, such that the transfected cells will produce sufficient amounts of the ribozyme to destroy the endogenous target messengers and inhibit translation. Since the ribozymes are catalytic/ unlike other antisense molecules, lower intracellular concentration is required for them to be effective.

*Inhibitory peptides*

As it is used herein, the term "inhibitory peptide" relates to those peptides capable of binding to LIF and inhibiting its activity as has been explained above, i.e., preventing LIF from being able to induce the activation of the JAK-STAT signaling pathway.

An example of an inhibitory peptide suitable for use in the present invention are pegylated variants of LIF as described in White et al. (J.Biol.Chem., 2007, Proc. Natl. Acad. Sci. USA, 104:19357-19362).

*Inhibitors of LIF receptor binding*

Inhibitors of cytokine receptor binding are those compounds which show affinity for the IL-6 type cytokine and are therefore capable of sequestering the cytokine and preventing the binding thereof to its physiological receptors. The inhibitory polypeptide is preferably a soluble form of the IL-6 type cytokine receptor (the so-called decoy receptors). In the particular case of LIF, it is possible to use a soluble variant of the LIF receptor or the LIF binding protein (LBP), a soluble form of the alpha LIF receptor found naturally and which has been found to be capable of effectively preventing the effects of LIF on the metabolism of proteoglycans in joint cartilage explants (Bell et al., 1997, J. Rheumatol. 24:2394).

*Inhibitory antibodies*

"Inhibitory antibody" and "neutralizing antibody" are used interchangeably to define any antibody which is capable of binding to LIF or binding to the receptor for LIF, preventing LIF from being able to induce the activation of the JAK-STAT signalling pathway. The antibodies can be prepared using any of the methods which are known for the person skilled in the art. Thus, polyclonal antibodies are prepared by means of immunization of an animal with the protein to be inhibited. Monoclonal antibodies can be prepared using the method described by Kohler, Milstein et al. (Nature, 1975, 256: 495). Although any other method known in the art would be equally suitable. Once antibodies with LIF binding capacity or with the capacity to bind to the receptors of said cytokine are identified, those which are capable of inhibiting the activity of this protein will be selected using the assay for the identification of inhibitory agents described above (Metz, 2007 mentioned above).

Therefore, in a more particular embodiment, the antibodies are inhibitory antibodies specific to LIF or antibodies blocking a LIF receptor.

Exemplary antibodies specific to LIF are described in US 5,654,157A, Kim et al., (J. Immunol. Meth., 156: 9-17, 1992), Alphonso et al., (J. Leukocyte Biology (Abstracts of the 28th National Meeting of the Society for Leukocyte Biology, vol. O, no. SP.2 (1991) (NY, N.E., p. 49) (Mabs D4.16.9, D25.1.4, and D62.3.2).

In a preferred embodiment, the antibody for use according to the present invention is an antibody which recognizes full length human LIF, but does not recognize a LIF fragment corresponding to amino acids 1 to 160. In another embodiment the antibody for use according to the present invention is an antibody which recognizes an epitope of human LIF comprised in the region corresponding to amino acids 160 to 202 of human LIF. In yet another embodiment, the antibody for use according to the present invention is an antibody which recognizes an epitope comprised in the regions selected from the following: a region corresponding to amino acids 160 to 180, a region corresponding to amino acids 170 to 190, a region corresponding to amino acids 180 to 200, a region corresponding to amino acids 182 to 202 of human LIF. In yet another embodiment, the antibody for use according to the present invention is an antibody which is competitively inhibited in its binding to human LIF by the monoclonal antibody produced by a hybridoma deposited on April 1st 2010 by Vall d'Hebron Institute of Oncology at the Deutsche Sammlung von Mikroorganismen und Zellkulturen GmbH (DSM ACC3054). In yet another embodiment, the antibody for use according to the present invention is the antibody which is produced by the hybridoma cell line DSM ACC3054, deposited on April 1st 2010 by Vall d'Hebron Institute of Oncology at the Deutsche Sammlung von Mikroorganismen und Zellkulturen GmbH, or an antigen-binding fragment thereof, a humanized form thereof or a chimeric form thereof.

In the present invention, the term "antibody" must be interpreted broadly and it includes polyclonal, monoclonal, multi-specific antibodies and fragments thereof (F(ab')2, Fab), etc. provided they are capable of specifically recognizing the antigen of interest, which in the context of the present invention is LIF or a receptor thereof. Examples of antibodies that can be used in the context of the present invention are, as non-limiting examples, polyclonal antibodies, monoclonal antibodies, recombinant antibodies, chimeric antibodies, humanized antibodies, completely human antibodies, etc.

The polyclonal antibodies are originally heterogeneous mixtures of antibody molecules produced in the serum of animals that have been immunized with an antigen. They also include monospecific polyclonal antibodies obtained from the heterogeneous mixtures, for example, by means of column chromatography with peptides of a single epitope of the antigen of interest.

A monoclonal antibody is a homogenous population of antibodies specific for a single epitope of the antigen. These monoclonal antibodies can be prepared by means of conventional techniques already described and routine for the skilled person.

A chimeric antibody is a monoclonal antibody constructed by means of the cloning or recombination of antibodies from different animal species. In a typical but non-limiting configuration of the invention, the chimeric antibody includes part of a monoclonal antibody, generally the variable fragment (Fv) including the sites for antigen recognition and binding, and the other part corresponding to a human antibody, generally the part including the constant region and the adjacent constant region.

A completely human antibody is an antibody or antibodies which have been produced in transgenic animals with a human immune system or by in vitro immunization of human immune cells (including both genetic and traditional immunization with or without adjuvants and pure or non-pure antigen; or by means of any method of exposure of the antigen to the immune system) or by means of native/synthetic libraries produced from human immune cells. These antibodies can be obtained and selected from transgenic animals (for example mice) in which human immunoglobulin genes have been cloned and which are immunized with the target antigen (in the present invention said antigen is LIF or a receptor thereof). These antibodies can be obtained by selecting human single-chain variable fragments (scFv) or antigen binding fragments (Fab) presented in phage displays and subsequent cloning and grafting in a human antibody or by means of any other production and display method known by the person skilled in the art, of the libraries generated by cloning the variable fragments of both strands and subsequent combination/mutation thereof to generate the antibody libraries.

A humanized antibody is a monoclonal antibody constructed by means of the cloning and grafting of the hypervariable complementarity determining regions (CDR) of a murine monoclonal antibody in a human antibody in replacement of their own hypervariable CDR regions.

In addition, in the context of the present invention, the term "antibody" also includes variants with an altered glycosylation pattern, as well as glycosylated or non-glycosylated antibody fragments, obtained from the protein or by means of recombinant technology, which can consist of (i) variable zones of the antibodies bound to one another by a binding peptide (scFv), (ii) the variable zone together with the CH1 constant of the heavy chain (Fd) bound to the light chain by means of cysteines or by means of binding peptides and disulfide bond (scFab), (iii) new variants, such as single heavy chains, or (iv) any modification made to the antibody fragments for the purpose of making them more similar, less immunogenic (humanized) or more stable in biological fluids and which in the context of the present invention, have the capacity to prevent LIF from performing their function (activity), i.e. inducing the activation of the JAK/STAT signaling pathway.

As the person skilled in the art will understand, the antibodies can be obtained by means of conventional genetic engineering or recombinant techniques, antibody production techniques, techniques for extraction and purification from biological fluids or tissues, or by any other conventional technique for obtaining proteins and antibodies which are widely known by the person skilled in the art. Illustrative non-limiting examples of techniques for the production of antibodies are: immunization techniques in animals, including transgenic animals for human immunoglobulin genes, production of monoclonal antibodies by means of hybridomas, production by means of antibody libraries which can be native, synthetic or derived from organisms immunized against the antigen of interest and which could be selected by means of very different display methods (phage display, ribosome display, etc.) and subsequently, by means of genetic engineering techniques they could be redesigned and expressed in vectors designed for the production of recombinant antibodies of different sizes, composition and structure.

In a particular embodiment, the agent for use of the invention in the treatment of ovarian, lung or colorectal cancer is an antibody specific for LIF, more preferably a LIF neutralising antibody. LIF neutralizing antibodies are commercially available and include, without limitation, goat polyclonal anti-human anti-LIF antibody from R&D Systems (catalog nos. AF-250-SP and AF-250-NA).

*Other compounds inhibiting the activity of LIF*

Other compounds with the capacity to inhibit the expression of an IL-6 type cytokine include aptamers and spiegelmers, which are single- or double-stranded D or L nucleic acids which bind specifically to the protein resulting from a modification of the biological activity thereof. Aptamers and spiegelmers have a length of between 15 and 80 nucleotides, and preferably between 20 and 50 nucleotides.

*Polypeptides with inhibitory activity of LIF*

Specifically, antagonists of LIF which could be useful in the context of the present invention are:

LIF variants presenting mutations in receptor binding sites which show a reduced affinity for same or which are capable of binding to only one of the chains of the receptor. Examples of said mutants include:

(i) the mutants described by Hudson et al. (J .Biol.Chem., 1996, 271:11971-11978),
(ii) the LIF variants described in WO2005030803 which have one or more mutations selected from the group of Q29A, G124R and N128A and which show a reduced affinity for the LIF receptor and for gp130. A high potency antagonist of LIF is the variant comprising MH35-BD/Q29A+G124R described by Fairlie/ W.D. et al. (J.Biol.Chem., 2004, 279:2125-2134),
(iii) The mutants described in WO9601319 characterized by having one or more substitutions in the receptor binding regions and, specifically, at positions 25-38, 150-160 or 161-180 with respect to the numbering of human LIF.
(iv) Soluble variants of the LIF receptor based on the primary structure and with the capacity of binding to LIF and preventing it from interacting with its native receptor on the cell surface, such as fusion proteins comprising part of the extracellular region of the LIF receptor and the gp130 ligand binding domain, as described by Metz; S. et al. (J.Biol.Chem., 2008, 283: 5985-5995).

The present invention relates to an agent capable of inhibiting the expression of LIF and/or capable of blocking the activity of LIF for use in the treatment of cancer, wherein said cancer is selected from ovarian cancer, lung cancer and colorectal cancer. In a particular embodiment, said cancer (ovarian, lung, colorectal cancer) is characterised by having increased LIF levels with respect to a reference value.

In one embodiment, the agent capable of inhibiting the expression of LIF and/or capable of blocking the activity of LIF for use according to the present invention is used for the treatment of patients characterized in that their tumors have increased LIF levels with respect to a reference value.

In one embodiment, the agent capable of inhibiting the expression of LIF and/or capable of blocking the activity of LIF for use according to the present invention is used for the treatment of patients which have been selected based on their tumors having have increased LIF levels with respect to a reference value.

In one embodiment, the agent capable of inhibiting the expression of LIF and/or capable of blocking the activity of LIF for use according to the present invention is used for the treatment of patients wherein the treatment is preceded by a selection of patients whose tumors have increased LIF levels with respect to a reference value. The selection of patients can be carried out according to the method for the selection of a cancer patient for a treatment based on a LIF inhibitor of the invention which is defined in detail below.

As the skilled person will understand, the quantification of expression levels of LIF in a sample can be performed as determination of the mRNA levels or as determination of the proteins levels. The quantification of the expression levels of the gene encoding LIF can be performed from the RNA resulting from the transcription of said gene (mRNA) or, alternatively, from the complementary DNA (cDNA) of said gene. Additionally, a step of extraction can be necessary for obtaining the total RNA, which can be performed by means of conventional techniques.

Virtually any conventional method can be used within the context of the invention to detect and quantify the levels of mRNA encoded by a gene encoding LIF or of its corresponding cDNA. By way of non-limiting illustration, the levels of mRNA encoded by said gene can be quantified by means of using conventional methods, for example, methods comprising the amplification of the mRNA and the quantification of the product of the amplification of said mRNA, such as electrophoresis and staining, or alternatively, by means of northern blot and the use of probes specific for the mRNA of the genes of interest or of their corresponding cDNA, mapping with the S1 nuclease, RT-LCR, hybridization, microarrays, etc., preferably, by means of quantitative real-time PCR using suitable sets of probes and primers. Similarly, the levels of the cDNA corresponding to said mRNA encoded by the gene encoding LIF can also be quantified by means of using conventional techniques; in this case, the method of the invention includes a step of synthesis of the corresponding cDNA by means of reverse transcription (RT) of the corresponding mRNA followed by amplification and quantification of the product of the amplification of said cDNA. Conventional methods for quantifying the expression levels can be found, for example, in Sambrook et al., 2001. "Molecular cloning: a Laboratory Manual", 3"d ed., Cold Spring Harbor Laboratory Press, N.E., Vol. 1-3. Thus, in a particular embodiment, the quantification of the expression levels of the gene encoding LIF comprises the quantification of the messenger RNA (mRNA) of said gene, a fragment of said mRNA, complementary DNA (cDNA) of said gene, a fragment of said cDNA, or mixtures thereof.

In another particular embodiment, the quantification of the expression levels of the gene encoding LIF is performed by means of a quantitative polymerase chain reaction.

In addition, the expression levels of the protein encoded by said gene encoding LIF, or the LIF protein, can also be quantified. As it is understood by the person skilled in the art, the expression level of a protein can be quantified by means of any conventional method. By way of non-limiting illustration, the levels of protein can be by means of the use of antibodies with the capacity to bind to said, proteins (or to fragments thereof containing an antigenic determinant) and the subsequent quantification of the complexes formed. The antibodies which are used in these assays may or may not be labelled. Illustrative examples of markers which can be used include radioactive isotopes, enzymes, fluorophores, chemiluminescent reagents, enzyme substrates or cofactors, enzyme inhibitors, particles, dyes, etc. There is a large variety of known assays which can be used in the present invention which use non-labelled antibodies (primary antibody) and labelled antibodies (secondary antibody) these techniques include Western-blot, ELISA (enzyme-linked immunosorbent assay), RIA (radioimmunoassay), competitive EIA (competitive enzyme immunoassay), DAS-ELISA (double-antibody sandwich ELISA), immunocytochemical and immunohistochemical techniques, techniques based on the use of, biochips or microarrays of proteins which include specific antibodies or assays based on colloidal precipitation in, formats such as dipsticks. Other ways to detect and quantify proteins include affinity chromatography techniques, ligand binding assays, etc. In another particular embodiment, the quantification of the levels of protein is performed by means of Western blot, immunohistochemistry or ELISA.

In a preferred embodiment, the levels of LIF in the sample are determined by immunohistochemistry. In a more preferred embodiment, the determination of the LIF levels by immunohistochemistry comprises the following steps:

- Sample fixation in formalin 4% overnight at room temperature,
- Embedding samples in paraffin,
- Incubate the slides at 60°C O/N,
- Deparaffinization and rehydratation: xylene + EtOH (100%, 90%, 70% and $H_2O$),
- Antigen Retrieval: DAKO solution pH6 (115°/3'),
- Let cool samples to Room Temperature (aprox. 20'),
- Peroxidase: 10' treatment (dilution 1:10),

- Washes TBS-T 1x (pH 7,6) 3x5'
- Blocking with 3% BSA in TBS-T1x, 30' for 1h at room temperature,
- Washes TBS-T 1x (pH 7,6) 3x5'
- Add anti-LIF antibody LIF (HPA018844, Atlas) 1:200 (green diluyent, DAKO) and maintain at 4° overnight in the humid chamber,
- Washes TBS-T 1x (pH 7,6) 3x5'
- Add secondary antibody for 20' at room temperature (envision, Dako)
- Washes TBS-T 1x (pH 7,6) 3x5'
- Developing: Maximum 20' before developing
- Counterstain with hematoxylin (20-30")
- Dehydratation: EtOH (70%, 90%, 100%) + xylene
- Mount slides

In another preferred embodiment, the determination of the expression levels of LIF can be carried out by means of the determination of the activity of said protein, since high expression levels generally result in a higher specific activity of said protein in a sample. Assays for determining the activity of LIF have been previously described in the context of assays for determining if a compound is an inhibitory agent of LIF.

In a particular embodiment of the agent for use according to the invention in the treatment of cancer selected from ovarian cancer, lung cancer and colorectal cancer, said cancer is characterised by having increased LIF levels

In one embodiment, increased LIF levels are defined by an absolute value. In a preferred embodiment, when the determination of LIF levels is carried out at the protein level, the LIF levels measured as a value of LIF score or as a value of H-score.

The LIF score is defined as the percentage (from 0% to 100%) of cells with a strong complete staining (3+) for LIF. For example, a LIF score of 5 means that 5% of tumor cells show a strong complete staining. Control samples show a LIF score of about 0, 1, 2, 3, 4 or 5, corresponding to samples from healthy individuals or to individuals not suffering from cancer, particularly not suffering from ovarian, lung or colorectal cancer. In a more particular embodiment, the cancer characterized by having high levels of LIF is characterized by having a LIF score of 6, 7, 8, 9, 10, 20, 30, 40, 50, 60, 70, 80, 90 or 100. In another embodiment, high LIF levels are defined as any LIF score which is above a given LIF-score. In a more particular embodiment, the cancer characterized by having high levels of LIF is characterized by having a LIF score greater than zero 1, 2, 3, 4, 5, 6, 7, 8, 9, 10, 20, 30, 40, 50, 60, 70, 80, 90, 95, 96, 97, 98 or, 99.

The term "H-score" relates to a particular value scale used to quantify LIF expression levels in a test sample. Based on the H score on a given sample, it can be concluded whether LIF levels are high or not. Control samples show a H score of 0, corresponding to samples from healthy individuals or to individuals not suffering from cancer, particularly not suffering from ovarian, lung or colorectal cancer or to healthy (non-tumoral) samples from the cancer patient. In a particular embodiment of the agent for use according to the present invention in the treatment of cancer selected from ovarian, lung and colorectal cancer, wherein the cancer is characterized by having increased levels of LIF with respect to a reference value, the levels of LIF are quantified by means of immunohistochemistry or immunohistofluorescence, said levels being measured as a value of H score. In a more particular embodiment, the cancer characterized by having high levels of LIF is characterized by having a H-score of at least 1, 10, 20, 30, 40, 50, 60, 70, 80, 90, 100, 120, 140, 160, 180, 200, 220, 240, 260, 280 or 300.

In another embodiment, the cancer is defined as having increased LIF levels when the levels are higher than a reference value. In this case, LIF expression levels are considered to be increased or to be higher than the reference value when it is at least at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than its reference value. Thus, increased levels of LIF with respect to a reference value relate to levels of LIF that are greater than a level of LIF considered as a reference value, by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150% or more.

The term "decreased levels" or "low levels", in relation to the expression level of LIF, relates to any level of expression of LIF in a sample lower the reference value. Thus, the LIF expression levels are considered to be decreased or to be lower than its reference value when it is at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than its reference value.

The term "similar levels" or "equal levels", in relation to the expression level of LIF, relates to any level of expression of LIF in a sample similar to that level of expression in a control sample or reference value. Thus, the levels are considered to be similar to those levels of the reference value when they differ in less than 5%, less than 4%, less than 3%, less than 2%, less than 1%, or less than 0,5%.

The term "reference value" or "reference level", as used in the context of the agent inhibiting LIF expression and/or blocking LIF activity for use according to the invention in the treatment of ovarian, lung or colorectal cancer, is understood as a predetermined criteria used as a reference for the determination of the levels of LIF in a test sample. The reference value or reference level can be an absolute value, a relative value, a value that has an upper or a lower limit, a range of values, an average value, a median value, a mean value, or a value as compared to a particular control or baseline value. A reference value can be based on an individual sample value, such as for example, a value obtained from a sample from the subject being tested, but at an earlier point in time. The reference value can be based on a large number of samples, such as from population of subjects of the chronological age matched group, or based on a pool of samples including or excluding the sample to be tested.

In a particular embodiment, the reference value corresponds to the LIF expression value in a sample obtained from a patient who is well documented from the clinical point of view, and who present no disease, particularly who is not suffering from cancer, particularly not suffering from ovarian cancer, lung cancer or colorectal cancer. Alternatively, the reference value corresponds to the LIF expression value obtained from a sample from a patient suffering from cancer, particularly suffering from ovarian cancer, lung cancer or colorectal cancer and which has been characterized as not responding to an anti-LIF treatment. Alternatively, the reference value corresponds to the LIF expression value obtained from healthy or non-tumoral tissue obtained from a patient suffering from cancer, particularly suffering from ovarian cancer, lung cancer or colorectal cancer, which has preferably been characterised as responding to an anti-LIF treatment. Preferably, the healthy tissue is the tissue from which the tumor has developed.

In a particular embodiment, the reference value corresponds to an average or mean LIF expression value determined from a pool of samples obtained from a group of patients who are well documented from the clinical point of view, and who present no disease, particularly who are not suffering from cancer, particularly not suffering from ovarian cancer, lung cancer or colorectal cancer. Alternatively, the reference value corresponds to an average or mean LIF expression value determined in a pool of samples obtained from a group of patients suffering from cancer, particularly suffering from ovarian cancer, lung cancer or colorectal cancer and which have been characterised as not responding to an anti-LIF treatment. Alternatively, the reference value corresponds to an average or mean LIF expression value obtained from a pool of healthy tissues or non-tumoral samples obtained from patients suffering from cancer, particularly suffering from ovarian cancer, lung cancer or colorectal cancer, which have preferably been characterised as responding to an anti-LIF treatment. In said samples, the expression levels can be determined, for example by means of the determination of the average expression level in a reference population. In the determination of the reference value, it is necessary to take into consideration some characteristics of the type of sample, such as age, gender, the physical state and the like of the patient. For example, the reference sample can be obtained from identical amounts of a group of at least 2, at least 10, at least 100 to more than 1000 individuals, such that the population is statistically significant.

It will be understood that the sample from which the reference value is obtained is usually the same type of sample which is used for determining LIF levels in the patient. For instance, if the determination of LIF levels in a patient is carried out in a biopsy from the tumor, the reference value will be obtained from non-tumoral sample of the same tissue. In some cases, the non-tumoral tissue may be obtained from the same tumor biopsy by obtaining those parts of the biopsy containing non-tumoral tissue.

As described above, the quantification of expression levels of LIF in a sample can be performed as determination of the mRNA levels or as determination of the proteins levels. In a preferred embodiment, the expression levels of LIF in a sample are determined as protein levels. In a more preferred embodiment, LIF protein levels are determined by means of western blotting, immunohistochemistry, immunohistofluorescence, mass spectrometry, mass spectrometry linked to immunoprecipitation or ELISA. In a more particular embodiment, the levels of LIF are quantified by means of immunohistochemistry or immunohistofluorescence.

Illustrative, non-limiting examples of samples according to the invention include different types of biological fluids, such as blood, serum, plasma, cerebrospinal fluid, peritoneal fluid, faeces, urine and saliva, as well as samples of tissues. The samples of biological fluids can be obtained by any conventional method like the samples of tissues; by way of illustration said samples of tissues can be samples of biopsies obtained by surgical resection. Preferably, the sample which is used for the determination of LIF is the same type of sample used for determining the reference value in case that the determination is done in relative terms. By way of a example, if the determination of the LIF levels are carried out in a sample of the tumor obtained after surgical resection, the reference value will also be a sample from a tumor obtained from a patient which has not responded to the treatment with LIF. If the sample is a biofluid, then the reference sample will also be determined in the same type of biofluid, e.g. blood, serum, plasma, cerebrospinal fluid.

In a particular embodiment of the agent for use according to the invention, the cancer is selected from ovarian cancer, lung cancer and colorectal cancer. In a more particular embodiment, the cancer is lung cancer, more particularly non-small cell lung cancer (NSCLC).

*Method of the invention for the design of a customized therapy for a cancer patient*

In another aspect, the present invention relates to an *in vitro* method for designing a customized therapy for a patient suffering from a cancer selected from ovarian cancer, lung cancer and colorectal cancer, comprising:

- quantifying the levels of LIF in a sample from said patient, and
- comparing said levels with a reference value,

wherein if the levels of LIF in said sample from said patient are equal or greater than the reference value, then an agent capable of inhibiting the expression and/or capable of blocking the activity of LIF is selected for administration to said patient.

The term "customized therapy", as used herein, also known as "personalized therapy" relates to the match of patients with treatments that are more likely to be effective and cause fewer side effects. In the context of the invention, the patient is a cancer patient, and the therapy is a therapy based on an agent capable of inhibiting the expression and/or capable of blocking the activity of LIF.

Thus, in a first step of the method of the invention for designing a customized therapy for a patient suffering from cancer, wherein said cancer is selected from ovarian cancer, lung cancer and colorectal cancer, the levels of LIF in a sample from said patient suffering from cancer are quantified. The quantification of the LIF levels are carried out as described above in the context of the therapeutic method of the invention.

In a particular embodiment, the patient for whom a customized therapy is to be designed is a lung cancer patient, more particularly a lung cancer patient suffering from non-small cell lung cancer (NSCLC).

Suitable samples according to the invention for determining the levels of LIF have been previously mentioned as well and incorporated herein. In a particular embodiment, the sample is a tissue sample or a biofluid. In a more particular embodiment, the tissue sample is a tumor sample. In a more particular embodiment, the biofluid is blood, serum or plasma.

Methods for quantifying LIF expression levels have been described previously in the context of the agent for use of the invention, and incorporated herein.

In a particular embodiment, the levels of LIF are quantified by means of western blotting, immunohistochemistry, immunohistofluorescence, mass spectrometry, mass spectrometry linked to immunoprecipitation or ELISA. More preferably, the levels of LIF are quantified by means of immunohistochemistry or immunohistofluorescence.

In a second step of the method of the invention for designing a customized therapy for a patient suffering from cancer, wherein said cancer is selected from ovarian cancer, lung cancer and colorectal cancer, the levels of LIF that have been quantified in a sample from the patient are compared to a reference value, wherein if the levels of LIF in the sample from the subject suffering from cancer are equal to or greater than zero, then an agent capable of inhibiting the expression and/or capable of blocking the activity of LIF is selected for administration to said patient.

The reference value according to the method of the invention for designing a customized therapy for a patient suffering from cancer is determined in the same manner as in the therapeutic method of the invention. References values according to the invention have been described previously in the context of the agent for use in the treatment of cancer of the invention, and incorporated herein.

In a particular embodiment, the levels of LIF are measured as a value of LIF score or as a value of H score. In a more particular embodiment, the levels of LIF are quantified by means of immunohistochemistry or immunohistofluorescence, and are measured as a value of LIF score or H score.

Agents capable of inhibiting the expression and/or capable of blocking the activity of LIF have been described above in the invention and incorporated herein. In a particular embodiment, the agent is selected from the group consisting of a siRNA specific for LIF, an antisense oligonucleotide specific for LIF and a ribozyme specific for LIF. In a particular alternative embodiment, is selected from the group consisting of an antibody specific for LIF, a polypeptide specific for LIF, an oligonucleotide specific for LIF and an inhibitor of LIF receptor binding specific for LIF. In a more particular embodiment, the antibody specific for LIF is a LIF neutralising antibody.

*Method of the invention for the selection of a cancer patient for a treatment based on a LIF inhibitor*

In a further aspect, the present invention relates to an *in vitro* method for selecting a patient suffering from a cancer selected from ovarian cancer, lung cancer and colorectal cancer to be treated with an agent capable of inhibiting the expression and/or capable of blocking the activity of LIF, comprising:

- quantifying the levels of LIF in a tumor sample from said patient, and
- comparing said levels with a reference value,

wherein if the levels LIF in said tumor sample from said patient are equal or greater than the reference value, then said patient is selected to receive treatment with an agent capable of inhibiting the expression and/or capable of blocking the activity of LIF.

In a first step of the method of the invention for selecting a patient suffering ovarian cancer, lung cancer and colorectal cancer to be treated with an agent capable of inhibiting the expression and/or capable of blocking the activity of LIF comprises quantifying the levels of LIF in a sample from said patient.

In a particular embodiment, the patient which is to be analyzed is a lung cancer patient, more particularly a lung cancer patient suffering from non-small cell lung cancer (NSCLC). In another embodiment, the patient is a ovarian cancer patient. In another embodiment, the patient is a colorectal cancer patient.

Suitable samples according to the invention for determining the levels of LIF have been previously mentioned as well and incorporated herein. In a particular embodiment, the sample is a tissue sample or a biofluid. In a more particular embodiment, the tissue sample is a tumor sample. In a more particular embodiment, the biofluid is blood, serum or plasma or a CSF sample.

Methods for quantifying LIF expression levels have been described previously in the context of the agent for use of the invention, and incorporated herein.

In a particular embodiment, the levels of LIF are quantified by means of western blotting, immunohistochemistry, immunohistofluorescence, mass spectrometry, mass spectrometry linked to immunoprecipitation or ELISA. More preferably, the levels of LIF are quantified by means of immunohistochemistry or immunohistofluorescence.

In a second step of the method of the invention for for selecting a patient suffering from a cancer selected from ovarian cancer, lung cancer and colorectal cancer to be treated with an agent capable of inhibiting the expression and/or capable of blocking the activity of LIF comprises comparing levels determined in the first step with a reference value. Reference values according to the method of the invention for selecting a patient for treatment with an agent capable of inhibiting the expression and/or capable of blocking the activity of LIF are determined in the same manner as in the therapeutic method of the invention. References values according to the invention have been described previously in the context of the agent for use in the treatment of cancer of the invention, and incorporated herein.

In a particular embodiment, the levels of LIF are measured as a value of LIF score or as a value of H score. In a more particular embodiment, the levels of LIF are quantified by means of immunohistochemistry or immunohistofluorescence, and are measured as a value of LIF score or H score.

Agents capable of inhibiting the expression and/or capable of blocking the activity of LIF have been described above in the invention and incorporated herein. In a particular embodiment, the agent is selected from the group consisting of a siRNA specific for LIF, an antisense oligonucleotide specific for LIF and a ribozyme specific for LIF. In a particular alternative embodiment, is selected from the group consisting of an antibody specific for LIF, a polypeptide specific for LIF, an oligonucleotide specific for LIF and an inhibitor of LIF receptor binding specific for LIF. In a more particular embodiment, the antibody specific for LIF is a LIF neutralising antibody.

The invention is described in detail below by means of the following examples which are to be construed as merely illustrative and not limitative of the scope of the invention.

EXAMPLES

*Heterogeneous transversal expression of LIF in cancer*

To assess the levels of LIF in cancer, samples from different tumor types were embedded in paraffin and stained by immunohistochemistry (IHC) with an anti-LIF antibody. LIF expression was quantified by immunohistochemistry using the H-Score method and represented in a graph.(

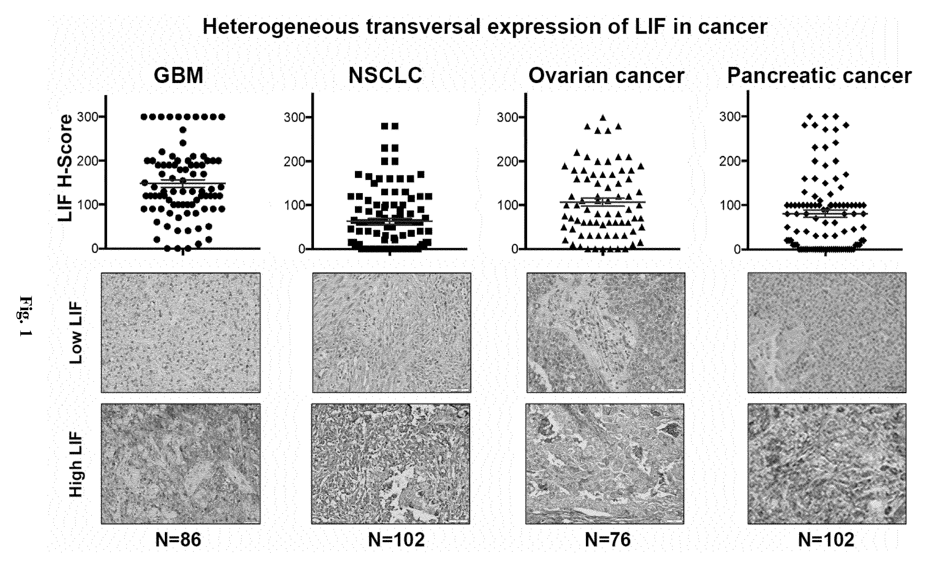

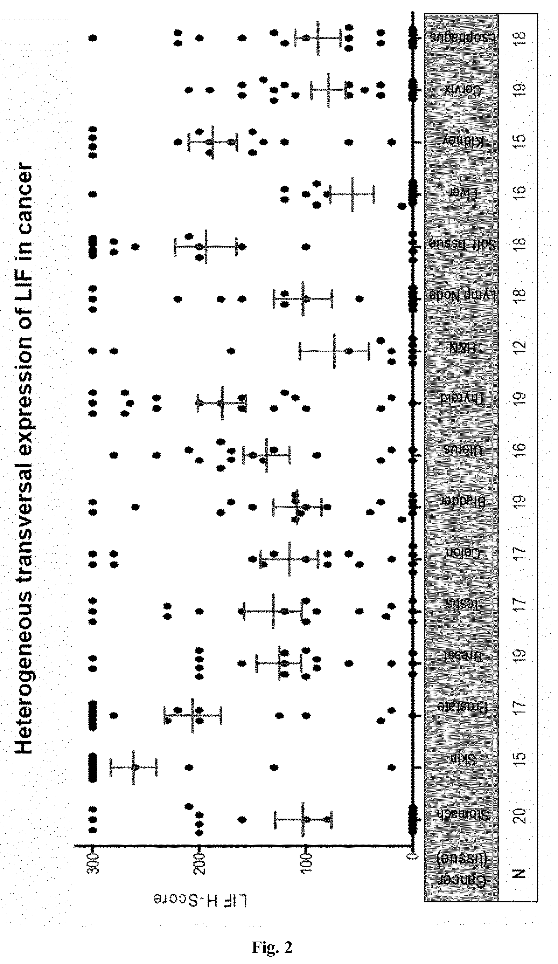

A wide variability in LIF expression was found in all indications. Importantly, tumors expressing extremely high levels of LIF protein were observed across the 20 cancer indications analyzed including glioblastoma (GBM), non-small cell lung carcinoma (NSCLC), ovarian cancer and pancreatic cancer (Figure 1 and 2).

*Determination of LIF score in glioma samples*

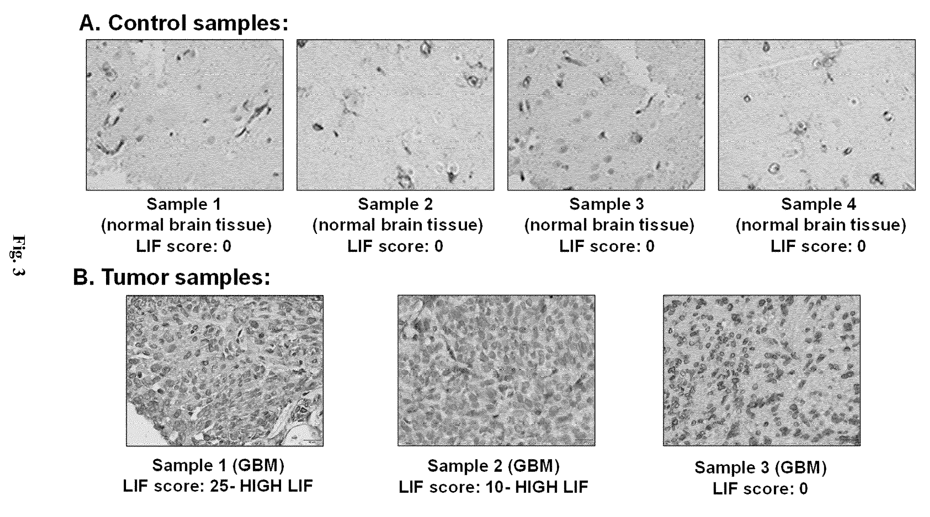

Control (Figure 3A) and tumor (Figure 3B) samples were fixed immediately after removal in a 10% buffered formalin solution for a maximum of 48 h at room temperature before being dehydrated and paraffin embedded under vacuum. Areas of representative tissue, away from necrotic foci, were identified on a hematoxylin-eosin-stained section. Determination of the levels of LIF protein by immunohistochemistry was carried out as follows:

1. Sample fixation in formalin 4% o/N, RT,
2. Sample are embedded in paraffin,
3. Incubate the slides at 60°C O/N,

4. Deparaffinization and rehydratation: xylene + EtOH (100% -> 90% -> 70% -> H2O)

5. Antigen Retrieval: DAKO solution pH6 (115°/3'). Let cool samples to Room Temperature (RT) (aprox 20')

6. Peroxidase: 10' treatment (dilution 1:10)

7. Washes TBS-T 1x (pH 7,6) 3x5'

8. Blocking with 3% BSA in TBS-T1x, 30'-1h, RT

9. Washes TBS-T 1x (pH 7,6) 3x5'

10. Primary antibody 4° O/N in the humid chamber LIF (HPA018844, Atlas) 1:200 (green diluyent, DAKO)

11. Washes TBS-T 1x (pH 7,6) 3x5'

12. Secondary antibody RT 20' (envision, Dako)

13. Washes TBS-T 1x (pH 7,6) 3x5'

14. Developing: Maximum 20' before developing

15. Counterstain with hematoxylin (20-30")

16. Dehydratation: EtOH (70% ->90% -> 100%) + xylene

17. Mount slides

For quantitative analysis of LIF protein score, the percentage and intensity of stained tumor cells was evaluated in representative high-power fields (x 400) on tissue sections using optical microscopy. The result was expressed as the percentage (from 0% to 100%) of cells with a strong complete staining. See Figure 3.

As can be observed, control samples have a LIF score of 0, whereas glioma samples varying LIF scores which range from 0 (sample 3), 10 (sample 2) or 25 (sample 1).

*Anti-LIF antibody inhibitory effect on tumor growth depends on the levels of LIF in said tumor*

Cells obtained from patients with GBM were stably infected with the Firefly luciferase gene for in vivo monitoring of bioluminescence. 100.000 cells were stereotactically inoculated into the corpus striatum of the right brain hemisphere (1mmanterior and 1.8mm lateral to the bregma; 2.5 mm intraparenchymal) of 9-week-old NOD/SCID mice (Charles River Laboratories).

Mice were treated or not with 15 mg/kg of the anti-LIF antibody intraperitoneally twice a week and tumor growth was monitored by bioluminescence. For the bioluminescence imaging, mice received an i.p. injection of 0.2 mL of 15 mg/mL D-luciferin under 1-2% inhaled isoflurane anesthesia. The bioluminescence signals were monitored using the IVIS system 2000 series (Xenogen Corp., Alameda, CA, USA) consisting of a highly sensitive cooled CCD camera. Living Image software (Xenogen Corp.) was used to grid the imaging data and integrate the total bioluminescence signals in each boxed region. Data were analyzed using the total photon flux emission (photons/second) in the regions of interest (ROI).

After one month treatment, mice were euthanized and the brain of the mice was removed. Brain samples were fixed immediately after removal in a 10% buffered formalin solution for a maximum of 48 hr at room temperature before being dehydrated and paraffin embedded under vacuum. Areas of representative tumor, away from necrotic foci, were identified on a hematoxylin-eosin-stained section. An anti-LIF antibody was used to stain the samples and determine the levels of LIF protein.

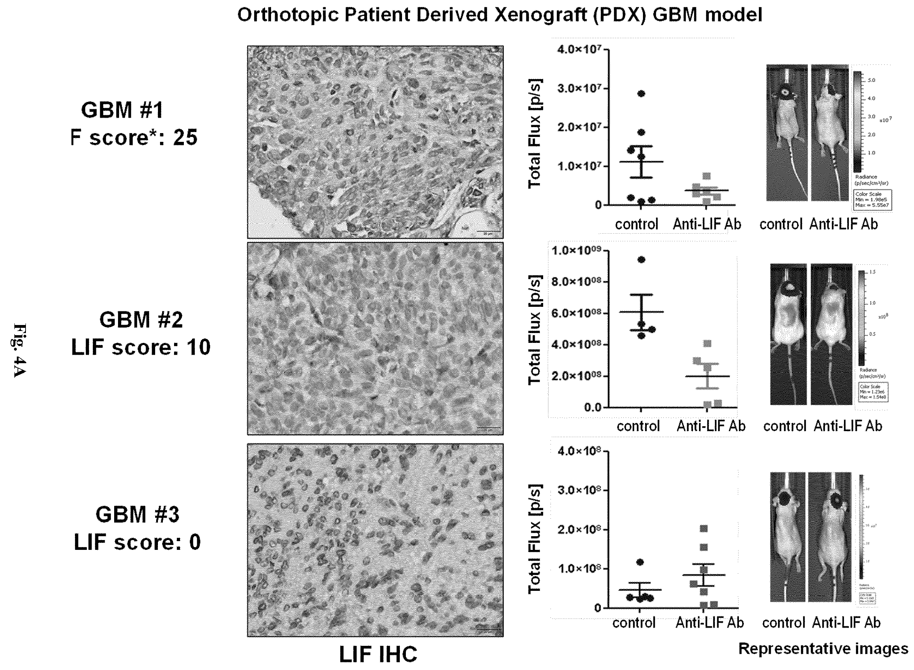

For quantitative analysis of LIF protein score, the percentage and intensity of stained tumor cells was evaluated in representative high-power fields (x 400) on tissue sections using optical microscopy. The result was expressed as the percentage (from 0% to 100%) of cells with a strong complete staining. See Figure 4A.

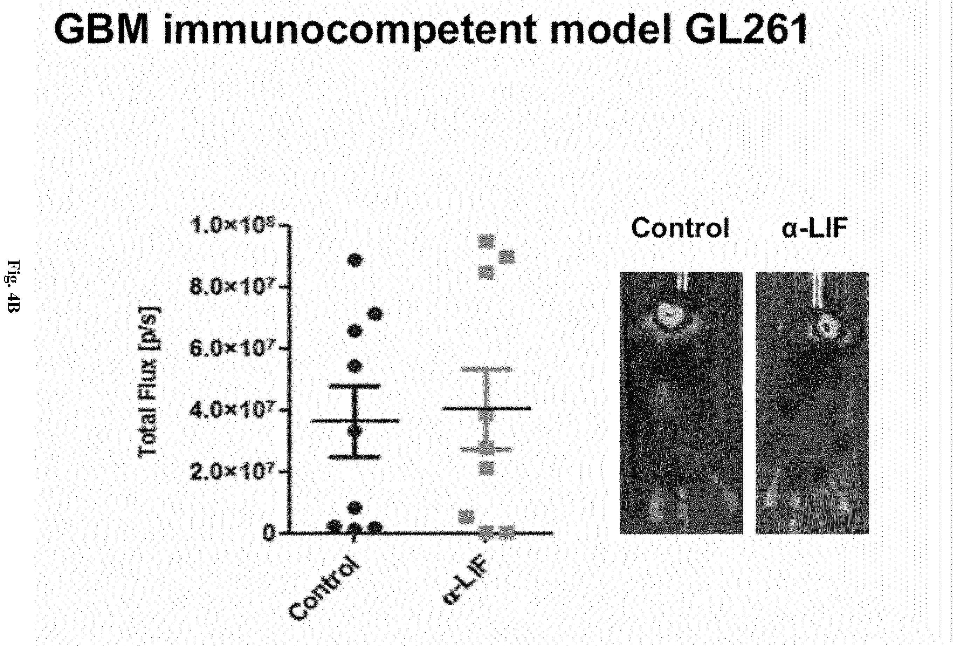

The results indicate that tumors with a LIF score at least above 10 (10% of cells with a strong complete staining) will be sensitive to the anti-LIF antibody treatment (see figure 4A), whereas tumors showing low LIF levels do not respond to anti-LIF therapy (figure 4B).

*Anti-LIF antibody promotes tumor growth inhibition in non-small cell lung cancer (NSCLC)*

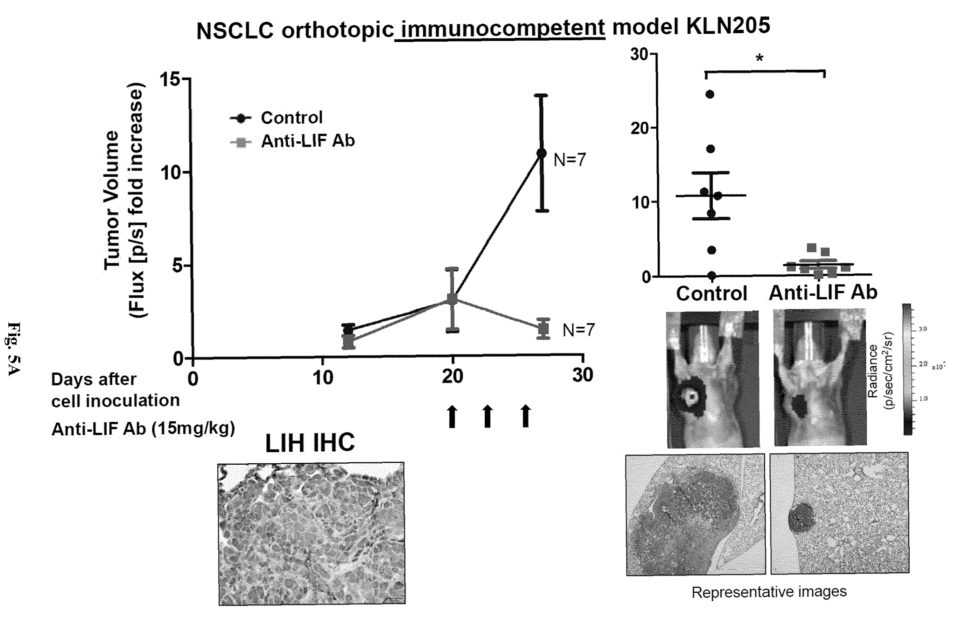

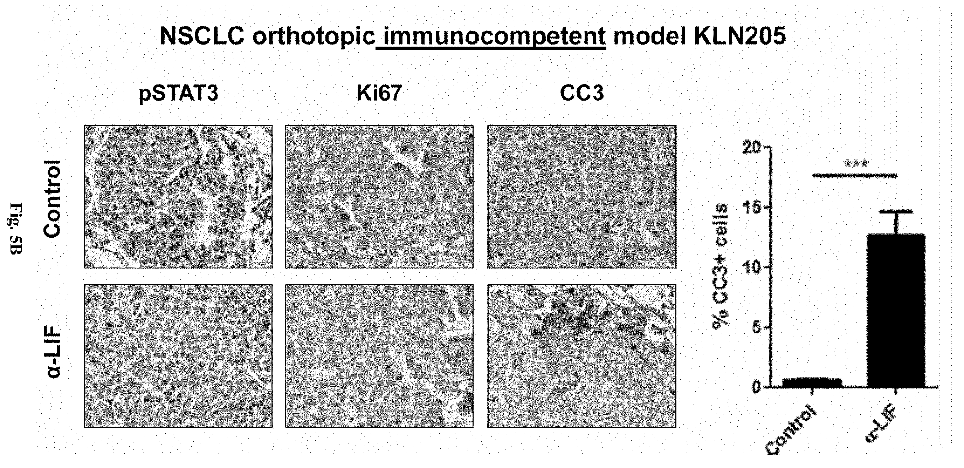

The murine non-small cell lung cancer (NSCLC) cell line KLN205 with high LIF levels was stably infected with lentivirus expressing the firefly luciferase gene for *in vivo* bioluminiscence monitoring. To develop the mouse model, KLN205 cells were orthotopically inoculated into the lung of immunocompetent syngeneic mice. See Figure 5.

Once the tumor was in an exponential growing phase, mice were treated or not with 15 mg/kg of the anti-LIF antibody intraperitoneally twice a week and tumor growth was monitored by bioluminescence. For the bioluminescence imaging, mice received an intraperitoneal injection of 0.2 mL of 15 mg/mL D-luciferin under 1-2% inhaled isoflurane anesthesia. The bioluminescence signals were monitored using the IVIS system 2000 series (Xenogen Corp., Alameda, CA, USA) consisting of a highly sensitive cooled CCD camera. Living Image software (Xenogen Corp.) was used to grid the imaging data and integrate the total bioluminescence signals in each boxed region. Data were analyzed using the total photon flux emission (photons/second) in the regions of interest (ROI). The results demonstrate that only 3 doses of anti-LIF antibody were sufficient to promote tumor regression. See Figure 5A.

Mice were euthanized and the lung of the mice was removed. Lung samples were fixed immediately after removal in a 10% buffered formalin solution for a maximum of 48 hr at room temperature before being dehydrated and paraffin embedded under vacuum. Areas of representative tumor, away from necrotic foci, were identified on a hematoxylin-eosin-stained section. Antibodies specific for phospho-STAT3 (p-STAT3), Ki67 and cleaved caspase 3 (CC3) were used to stain the samples and determine the levels of the pSTAT3 (readout of LIF pathway), Ki67 (proliferation marker) and CC3 (apoptosis marker). Representative images are shown. Scale bar, 20 $\mu$m (middle panels). Percentage of CC3+ cells was calculated. Data are presented as mean $\pm$ SEM (right panels). Results show that treatment with anti-LIF antibody inhibits the LIF pathway (pSTAT3 levels) and decreases cell proliferation (Ki67) while increasing apoptosis (CC3 staining).See Figure 5B.

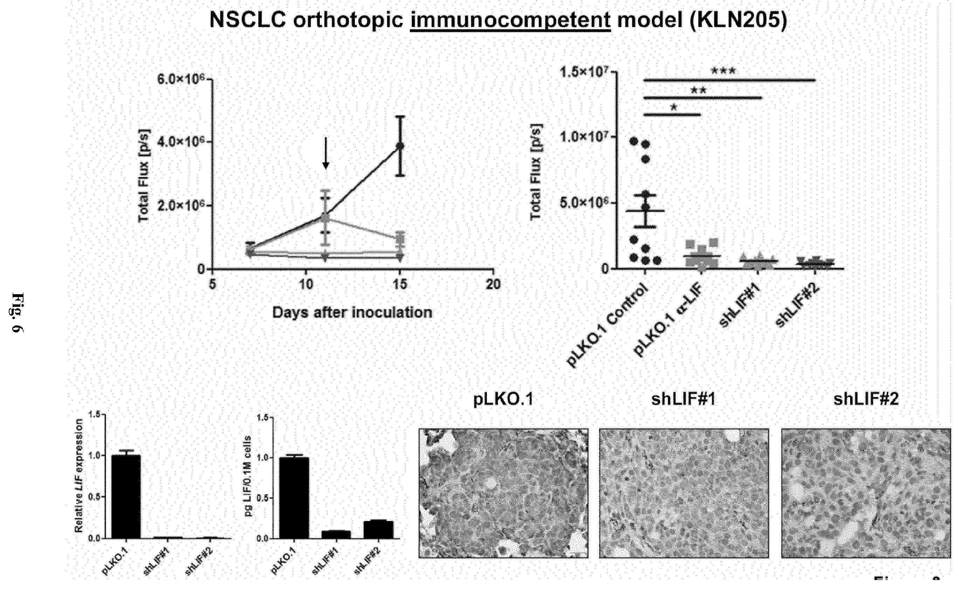

In order to confirm that the effect on tumor growth *in vivo* was due to a specific effect of the anti-LIF antibody on the LIF pathway, cells were infected with a lentivirus expressing a short hairpin against LIF (shLIF) or a sh Control in the KLN205 cells. Levels of LIF in KLN205 cells infected with the shLIF lentivirus were lower, as analyzed by mRNA levels (real time PCR) and protein levels (ELISA). See Figure 6.

KLN205 cells expressing the short hairpins were inoculated in the lung of syngeneic mice and tumor growth was monitored with a CCD camera. KLN205 tumors from cells infected with the shLIF are smaller than the tumors infected with the shControl, indicating that the tumorigenic activity depends on LIF. When the tumors were stained by IHC, it was confirmed that the levels of LIF in the tumors infected with the shLIF were lower. In addition, when the mice with KLN205 shLIF tumors were treated with anti-LIF antibody 15 mg/kg twice a week, an additional reduction of the tumor was not observed. These results indicate that anti-LIF antibody is specifically targeting LIF and that the therapeutic effect observed with the anti-LIF antibody was not due to an off-target effect of anti-LIF antibody. Prior to inoculation, LIF transcript levels were determined by qRT-PCR analysis and secreted LIF protein levels were determined by ELISA. Data are presented as mean $\pm$ SD. Immunohistochemistry for LIF was also performed in diaphragm tumors. These results confirm that the cells infected with shLIF have a decreased expression of LIF. Representative images are shown. Scale bar, 20 $\mu$m (right panel). See Figure 6.

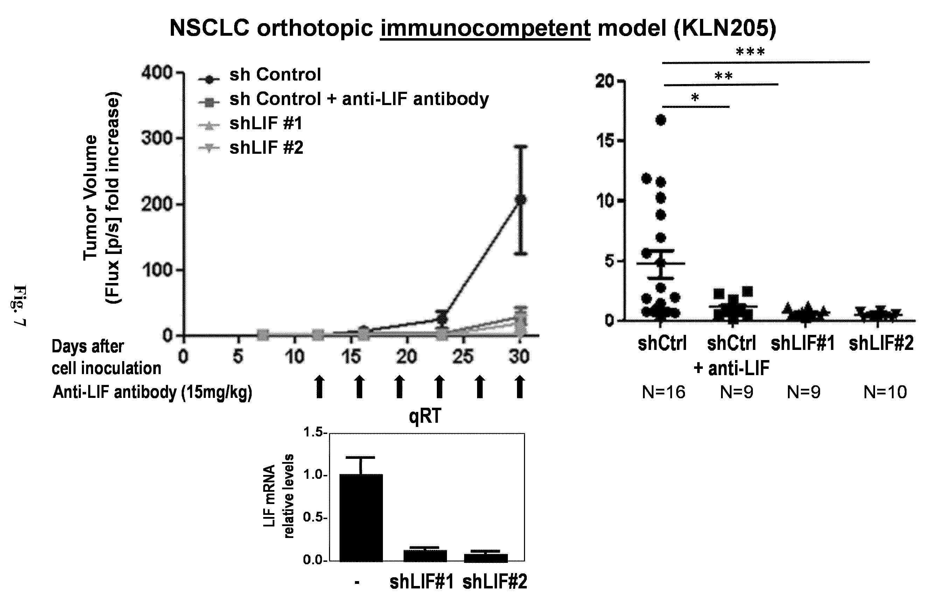

An additional experiment was performed to confirm the role of LIF in the NSCLC model and the therapeutic effect of anti-LIF antibody. In this experiment 2 independent short hairpins targeting LIF were used. Both short hairpins efficiently decreased the levels of LIF mRNA. Prior to inoculation, LIF transcript levels were determined by qRT-PCR analysis and secreted LIF protein levels were determined by ELISA. Data are presented as mean $\pm$ SD. See Figure 7. KLN205 cells infected with the two independent shLIF or shControl lentivirus were inoculated in the lung of syngeneic mice. When mice inoculated with tumors expressing the shControl were treated with anti-LIF antibody 15 mg/kg twice a week, a clear decrease in tumor growth was observed, confirming the therapeutic effect on anti-LIF antibody. See Figure 7.

In addition, tumors with cells infected with both shLIF produced smaller tumors confirming that tumor growth depends on LIF pathway. Immunohistochemistry for LIF was also performed in diaphragm tumors. Representative images are shown. Scale bar, 20 $\mu$m (right panel).

*Anti-LIF antibody promotes tumor growth inhibition in glioblastoma*

U251 GBM cells with high levels of LIF as measured by IHC infected with the firefly luciferase gene, were stereotactically inoculated into the corpus striatum of the right brain hemisphere (1 mm anterior and 1.8 mm lateral to the bregma; 2.5 mm intraparenchymal) of 9-week-old NOD/SCID mice (Charles River Laboratories). These mice are immunodeficient but have active macrophages.

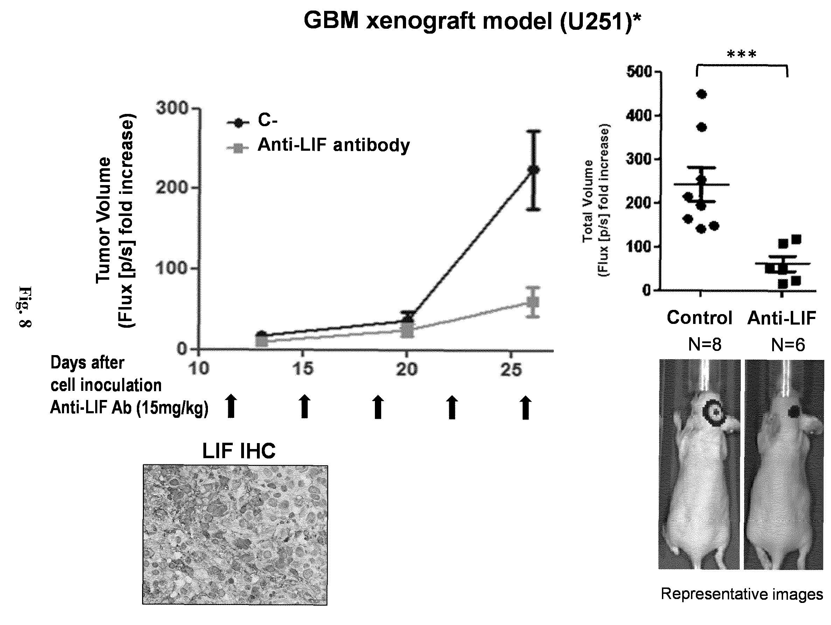

Seven days after cell inoculation, mice were treated with 15 mg/kg of the anti-LIF antibody intraperitoneally twice a week and tumor growth was monitored by bioluminiscence. Anti-LIF antibody treatment inhibited tumor growth. See Figure 8.

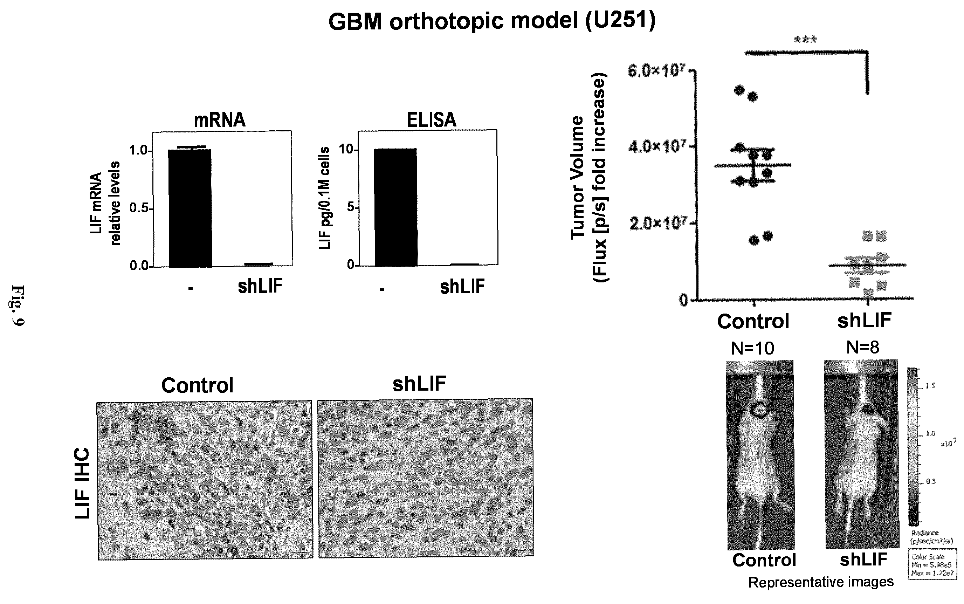

In order to confirm that the effect on tumor growth *in vivo* was dependent on the LIF pathway, U251 cells were infected with a lentivirus expressing a short hairpin against LIF (shLIF) or short hairpin Control. Levels of LIF in U251 cells infected with the shLIF were lower, as analyzed by mRNA levels (real time PCR) and protein levels (ELISA). Tumor growth was monitored with a CCD camera and the results demonstrate that U251 tumors from cells infected with the shLIF are smaller, indicating that the tumorigenic activity depends on LIF. In addition, IHC confirmed that tumors with the shLIF had lower levels of LIF protein expression by IHC. See Figure 9.

*Anti-LIF antibody promotes tumor growth inhibition in ovarian cancer*

Murine ovarian cancer cells from cell line ID8 with high LIF levels were inoculated into the peritoneum of immunocompetent syngeneic mice.

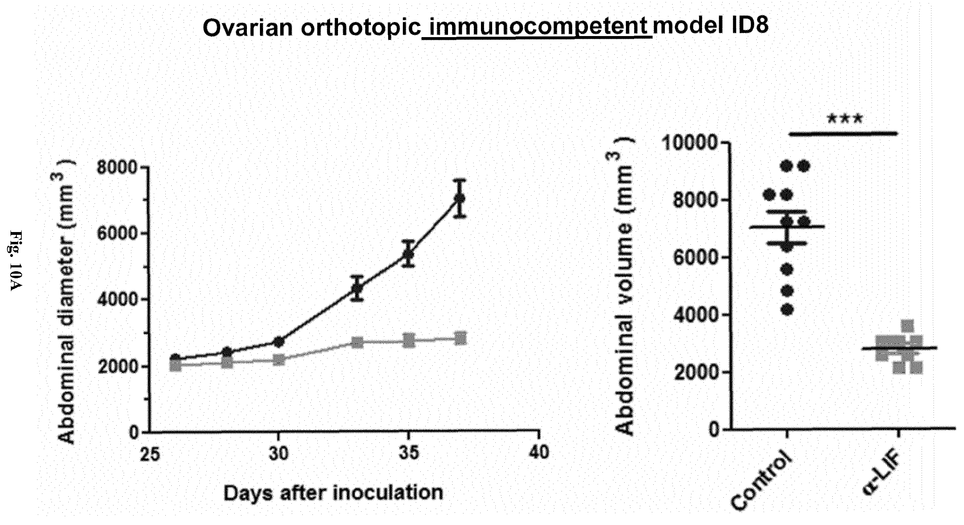

Mice were treated with 15 mg/kg of the anti-LIF antibody intraperitoneally twice a week and the waist of the mice was measured as a measure of ascites and a surrogate marker of tumor growth. Mice treated with anti-LIF antibody had a decreased waist perimeter indicating that anti-LIF antibody inhibited tumor growth. See Figure 10A.

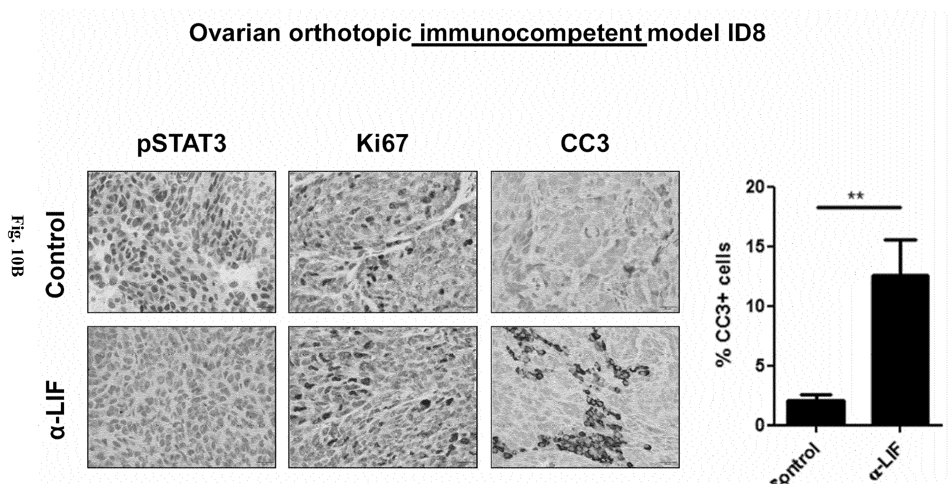

Mice were euthanized and the tumor of the mice was removed. Tumor samples were fixed immediately after removal in a 10% buffered formalin solution for a maximum of 48 hr at room temperature before being dehydrated and paraffin embedded under vacuum. Areas of representative tumor, away from necrotic foci, were identified on a hematoxylin-eosin-stained section. Antibodies specific for p-STAT3, Ki67 and cleaved caspase 3 (CC3) were used to stain the samples and determine the levels of the pSTAT3 (readout of LIF pathway), Ki67 (proliferation marker) and CC3 (apoptosis marker) were performed in all models. Representative images are shown. Scale bar, 20 $\mu$m (middle panels). Percentage of CC3+ cells was calculated. Data are presented as mean $\pm$ SEM (right panels). Results show that treatment with anti-LIF antibody *in vivo* inhibits the LIF pathway (pSTAT3 levels) and decreases cell proliferation (Ki67) while increasing apoptosis (CC3 staining). See Figure 10B.

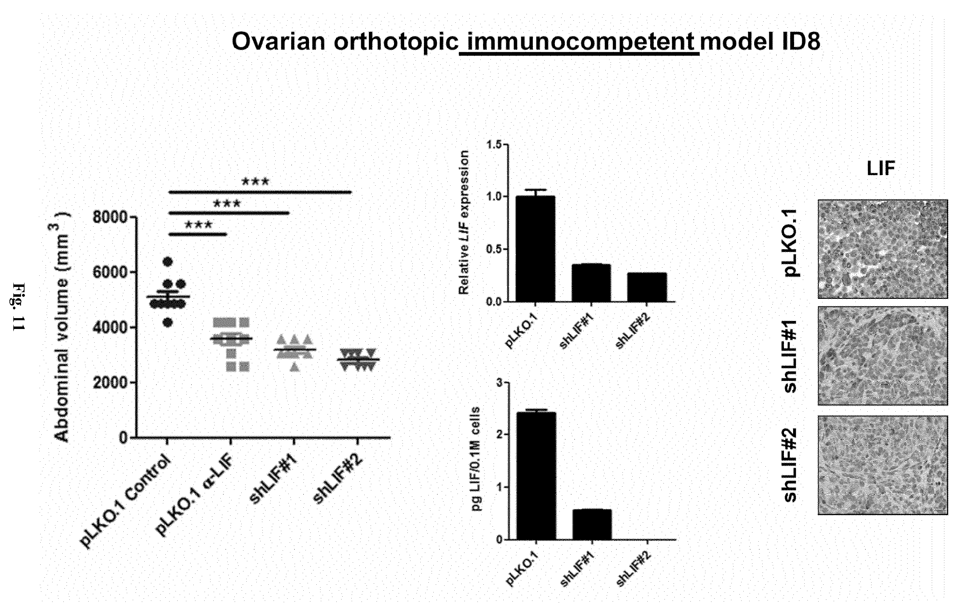

In order to confirm that the effect on tumor growth *in vivo* was dependent on the LIF pathway, ID8 cells were infected with a lentivirus expressing two independent short hairpin against LIF (shLIF) or short hairpin Control. Levels of LIF in ID8 cells infected with the shLIF were lower. Prior to inoculation, LIF transcript levels were determined by qRT-PCR analysis and secreted LIF protein levels were determined by ELISA. Data are presented as mean $\pm$ SD. Waist perimeter was measured as a measure of the ascites and a surrogate marker or tumor growth. Results demonstrate that ID8 tumors from cells infected with the shLIF are smaller, indicating that the tumorigenic activity depends on LIF. Immunohistochemistry for LIF was also performed in tumors confirming that LIF levels in cells infected with the short hairpins are lower. Representative images are shown. Scale bar, 20 $\mu$m (right panel). See Figure 11.

*Anti-LIF antibody promotes tumor growth inhibition in colorectal cancer*

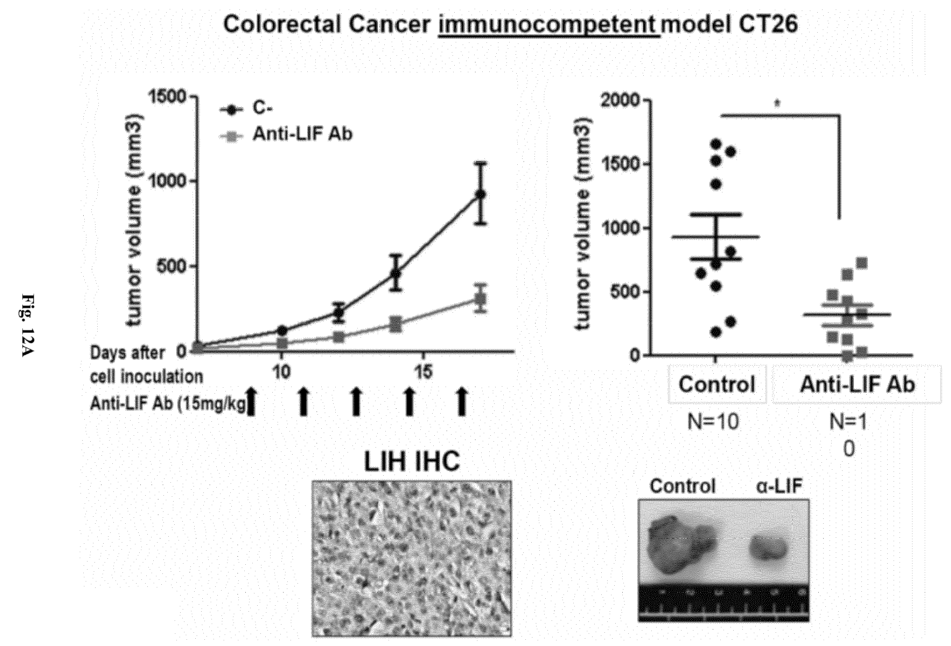

Murine colorectal cancer cells CT26 were subcutaneously inoculated into the right flank of syngeneic mice. On day 5, anti-LIF antibody treatment with 15 mg/kg twice a week given intraperitoneally was started. Tumor growth was assessed by caliper measurement. Tumor volume (mm$^3$) was calculated by the formula $\pi/6$ x larger diameter x (smaller diameter)$^2$. Results are presented as mean $\pm$ s.d. Anti-LIF antibody treatment decreased tumor growth. Mice were euthanized and the tumor of the mice was removed and pictures were taken from representative tumors. See Figure 12A.

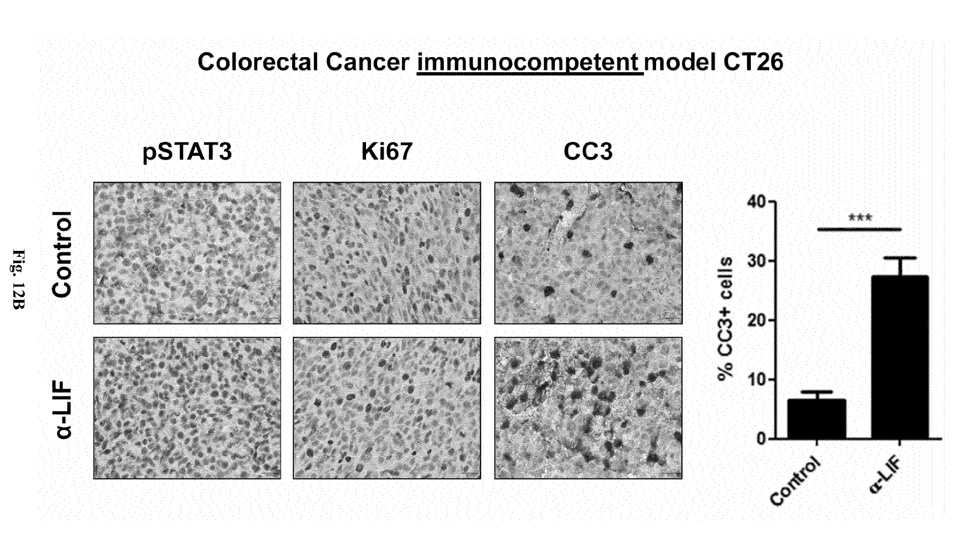

Mice were euthanized and the tumor of the mice was removed. Tumor samples were fixed immediately after removal in a 10% buffered formalin solution for a maximum of 48 hr at room temperature before being dehydrated and paraffin embedded under vacuum. Areas of representative tumor, away from necrotic foci, were identified on a hematoxylin-eosin-stained section. Antibodies specific for pSTAT3, Ki67 and cleaved caspase 3 (CC3) were used to stain the samples and determine the levels of the pSTAT3 (readout of LIF pathway), Ki67 (proliferation marker) and CC3 (apoptosis marker) were performed in all models. Representative images are shown. Scale bar, 20 $\mu$m (middle panels). Percentage of CC3+ cells was calculated. Data are presented as mean $\pm$ SEM (right panels). Results show that MSC-1 treatment in vivo inhibits the LIF pathway (pSTAT3 levels) and decreases proliferation while increasing apoptosis (CC3 staining). See Figure 12B.

**Claims**

1. An agent capable of inhibiting the expression and/or capable of blocking the activity of leukemia inhibitory factor (LIF) for use in the treatment of a cancer selected from ovarian cancer, lung cancer and colorectal cancer.

2. The agent for use according to claim 1, wherein said cancer is **characterised by** having high LIF levels.

3. An *in vitro* method for designing a customised therapy for a patient suffering from a cancer selected from ovarian cancer, lung cancer and colorectal cancer, comprising:

   (i) quantifying the levels of LIF in a sample from said patient, and
   (ii) comparing said levels with a reference value,
   wherein if the levels of LIF in said sample from said patient are equal or greater than the reference value, then an agent capable of inhibiting the expression and/or capable of blocking the activity of LIF is selected for administration to said patient.

4. An *in vitro* method for selecting a patient suffering from a cancer selected from ovarian cancer, lung cancer and colorectal cancer to be treated with an agent capable of inhibiting the expression and/or capable of blocking the activity of LIF, comprising:

(i) quantifying the levels of LIF in a sample from said patient, and

(ii) comparing said levels with a reference value,

wherein if the levels LIF in said sample from said patient are equal or greater than the reference value, then said patient is selected to receive treatment with an agent capable of inhibiting the expression and/or capable of blocking the activity of LIF.

5. The method according to any of claims 3 or 4, wherein the sample is a tissue sample or a biofluid.

6. The method according to claim 5, wherein the tissue sample is a tumor sample.

7. The method according to claim 5, wherein the biofluid is blood, serum or plasma.

8. The agent for use according to any of claims 1 or 2, or the method according to any of claims 3 to 7, wherein the levels of LIF are quantified by means of western blotting, immunohistochemistry, immunohistofluorescence, mass spectrometry, mass spectrometry linked to immunoprecipitation or ELISA.

9. The agent for use according to claim 8, or the method according to claim 5, wherein the levels of LIF are quantified by means of immunohistochemistry or immunohistofluorescence and are measured as a value of LIF-score or H-score.

10. The agent for use according to claim 9, wherein the cancer is **characterised by** having a LIF-score equal to or greater than zero or an H-score equal or greater than zero.

11. The method according to claim 10, wherein the reference value is a LIF-score equal or greater than zero or an H-score equal or greater than zero.

12. The agent for use according to any of claims 1 or 2, or the method according to any of claims 3 to 9 and 11, wherein the agent capable of inhibiting the expression of LIF is selected from the group consisting of a siRNA specific for LIF, an antisense oligonucleotide specific for LIF and a ribozyme specific for LIF.

13. The agent for use according to any of claims 1 or 2, or the *in vitro* method according to any of claims 3 to 9 and 11, wherein the agent capable of blocking the activity of LIF is selected from the group consisting of an antibody specific for LIF, a polypeptide specific for LIF, an oligonucleotide specific for LIF and an inhibitor of LIF receptor binding specific for LIF.

14. The agent for use according to claim 13, or the method according to claim 13, wherein said antibody specific for LIF is a LIF neutralising antibody.

15. The agent for use according to any of claims 1 or 2, or the method according to any of claims 3 to 9 and 11 to 14, wherein the lung cancer is non-small cell lung cancer (NSCLC).

Fig. 1

Fig. 2

A. Control samples:

Sample 1
(normal brain tissue)
LIF score: 0

Sample 2
(normal brain tissue)
LIF score: 0

Sample 3
(normal brain tissue)
LIF score: 0

Sample 4
(normal brain tissue)
LIF score: 0

B. Tumor samples:

Sample 1 (GBM)
LIF score: 25 - HIGH LIF

Sample 2 (GBM)
LIF score: 10 - HIGH LIF

Sample 3 (GBM)
LIF score: 0

**Fig. 3**

**Fig. 4A**

Fig. 4B

Fig. 5A

Fig. 5B

Fig. 6

28

Fig. 7

**GBM xenograft model (U251)***

Fig. 8

# GBM orthotopic model (U251)

Fig. 9

EP 3 173 483 A1

Ovarian orthotopic immunocompetent model ID8

**Fig. 10A**

Fig. 10B

EP 3 173 483 A1

Fig. 11

34

**Fig. 12A**

Colorectal Cancer <u>immunocompetent</u> model CT26

Fig. 12B

36

EP 3 173 483 A1

Europäisches
Patentamt

European
Patent Office

Office européen
des brevets

## EUROPEAN SEARCH REPORT

Application Number

EP 15 38 2590

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2015/040243 A2 (INSERM INST NAT DE LA SANTÉ ET DE LA RECH MÉDICALE [FR]; CENTRE NAT RE) 26 March 2015 (2015-03-26)<br>* X for inventions 2 & 3 *<br>----- | 1-12,14, 15 | INV.<br>C12N15/113<br>C07K16/22<br>C07K16/24<br>A61K39/00<br>A61K31/7088<br>A61P35/00 |
| A | US 2015/133376 A1 (CHANG YU-SUN [TW] ET AL) 14 May 2015 (2015-05-14)<br>----- | 1-14 | |
| A | WO 2011/124566 A1 (FUNDACIO PRIVADA INST D INVESTIGACIO ONCOLOGICA DE VALL HEBRON VHIO [E] 13 October 2011 (2011-10-13)<br>----- | 1-14 | |
| A | JUNG EUN SHIN ET AL: "Epigenetic up-regulation of leukemia inhibitory factor (LIF) gene during the progression to breast cancer",<br>MOLECULES AND CELLS,<br>vol. 31, no. 2,<br>3 December 2010 (2010-12-03), pages 181-189, XP055244105,<br>KR<br>ISSN: 1016-8478, DOI: 10.1007/s10059-011-0020-z<br>----- | 1-14 | |
| A | ESTROV Z ET AL: "LEUKEMIA INHIBITORY FACTOR BINDS TO HUMAN BREAST CANCER CELLS AND STIMULATES THEIR PROLIFERATION",<br>JOURNAL OF INTERFERON AND CYTOKINE RESEARCH, MARY ANN LIEBERT, NEW YORK, NY, US,<br>vol. 15, no. 10,<br>1 October 1995 (1995-10-01), pages 905-913, XP001037939,<br>ISSN: 1079-9907<br>-----<br><br>-/-- | 1-14 | TECHNICAL FIELDS SEARCHED (IPC)<br><br>C12N<br>C07K<br>A61K |

The present search report has been drawn up for all claims

| Place of search | Date of completion of the search | Examiner |
|---|---|---|
| The Hague | 7 November 2016 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 15 38 2590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KAMOHARA HIDENOBU ET AL: "Leukemia inhibitory factor functions as a growth factor in pancreas carcinoma cells: Involvement of regulation of LIF and its receptor expression", INTERNATIONAL JOURNAL OF ONCOLOGY, DEMETRIOS A. SPANDIDOS ED. & PUB, GR, vol. 30, no. 4, 1 April 2007 (2007-04-01), pages 977-983, XP009136730, ISSN: 1019-6439 ----- | 1-14 | |
| A | LIU J ET AL: "Expression of leukemia-inhibitory factor as an autocrinal growth factor in humanmedulloblastomas", JOURNAL OF CANCER RESEARCH AND CLINICAL ONCOLOGY, SPRINGER INTERNATIONAL, BERLIN, DE, vol. 125, no. 8-9, 1 August 1999 (1999-08-01), pages 475-480, XP002980338, ISSN: 0171-5216, DOI: 10.1007/S004320050304 ----- | 1-14 | |
| A | WYSOCZYNSKI MARCIN ET AL: "An in vitro and in vivo evidence that downregulation of leukemia inhibitory factor (LIF) receptor (LIF-R) decreases the metastatic potential of human rhabdomyosarcoma (RMS) cells.", BLOOD, vol. 108, no. 11, Part 1, November 2006 (2006-11), page 724A, XP002756746, & 48TH ANNUAL MEETING OF THE AMERICAN-SOCIETY-OF-HEMATOLOGY; ORLANDO, FL, USA; DECEMBER 09 -12, 2006 ISSN: 0006-4971 ----- -/-- | 1-14 | TECHNICAL FIELDS SEARCHED (IPC) |

The present search report has been drawn up for all claims

| Place of search | Date of completion of the search | Examiner |
|---|---|---|
| The Hague | 7 November 2016 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 15 38 2590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ALBRENGUES J ET AL: "LIF mediates proinvasive activation of stromal fibroblasts in cancer", CELL REPORTS, ELSEVIER INC, US, vol. 7, no. 5, 12 June 2014 (2014-06-12), pages 1664-1678, XP002733832, ISSN: 2211-1247, DOI: 10.1016/J.CELREP.2014.04.036 [retrieved on 2014-05-22] ----- | 1-14 | |
| A | B. W. C. TSE ET AL: "Antibody-based immunotherapy for ovarian cancer: where are we at?", ANNALS OF ONCOLOGY., vol. 25, no. 2, 26 November 2013 (2013-11-26), pages 322-331, XP055266783, NL ISSN: 0923-7534, DOI: 10.1093/annonc/mdt405 ----- | 12-14 | |
| A | F YE ET AL: "Expression of leukaemia inhibitory factor in epithelial ovarian carcinoma: correlation with clinical characteristics", HISTOPATHOLOGY., vol. 53, no. 2, 1 August 2008 (2008-08-01), pages 224-228, XP055266718, GB ISSN: 0309-0167, DOI: 10.1111/j.1365-2559.2008.03068.x ----- -/-- | 12-14 | TECHNICAL FIELDS SEARCHED (IPC) |

The present search report has been drawn up for all claims

| Place of search | Date of completion of the search | Examiner |
|---|---|---|
| The Hague | 7 November 2016 | Piret, Bernard |

EPO FORM 1503 03.82 (P04C01)

page 3 of 7

Europäisches
Patentamt

European
Patent Office

Office européen
des brevets

# EUROPEAN SEARCH REPORT

Application Number

EP 15 38 2590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | XIAOFENG ZHAO ET AL: "Human epithelial ovarian carcinoma cell-derived cytokines cooperatively induce activated CD4 + CD25 - CD45RA + naïve T cells to express forkhead box protein 3 and exhibit suppressive ability in vitro", CANCER SCIENCE, vol. 100, no. 11, 1 November 2009 (2009-11-01), pages 2143-2151, XP055266869, JP ISSN: 1347-9032, DOI: 10.1111/j.1349-7006.2009.01286.x ----- | 12-14 | |
| A | E Dorothé ET AL: "Tumor-associated leukemia inhibitory factor and IL-6 skew monocyte differentiation into tumor-associated macrophage-like cells", , 15 December 2007 (2007-12-15), XP055249144, DOI: 10.1182/blood-2007-02- Retrieved from the Internet: URL:http://www.bloodjournal.org/content/110/13/4319.full.pdf [retrieved on 2016-02-10] ----- -/-- | 1-14 | |

TECHNICAL FIELDS
SEARCHED (IPC)

The present search report has been drawn up for all claims

| Place of search | Date of completion of the search | Examiner |
|---|---|---|
| The Hague | 7 November 2016 | Piret, Bernard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 15 38 2590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Y. CHEN ET AL: "Gprc5a Deletion Enhances the Transformed Phenotype in Normal and Malignant Lung Epithelial Cells by Eliciting Persistent Stat3 Signaling Induced by Autocrine Leukemia Inhibitory Factor", CANCER RESEARCH, vol. 70, no. 21, 19 October 2010 (2010-10-19), pages 8917-8926, XP055264999, US ISSN: 0008-5472, DOI: 10.1158/0008-5472.CAN-10-0518 ----- | 1-15 | |
| A | FU J ET AL: "Effect of interleukin-6 on the growth of human lung cancer cell line", CHINESE MEDICAL JOURNAL / ZHONGHUA YIXUE ZAZHI YINGWEN BAN, CHINESE MEDICAL ASSOCIATION, BEIJING, CN, vol. 111, no. 3, 1 March 1998 (1998-03-01) , pages 265-268, XP008182098, ISSN: 0366-6999 ----- | 1-15 | |
| A | EDWARD B. GARON: "Current Perspectives in Immunotherapy for Non-Small Cell Lung Cancer", SEMINARS IN ONCOLOGY, vol. 42, 1 October 2015 (2015-10-01), pages S11-S18, XP055247767, US ISSN: 0093-7754, DOI: 10.1053/j.seminoncol.2015.09.019 ----- -/-- | 1-15 | TECHNICAL FIELDS SEARCHED (IPC) |

The present search report has been drawn up for all claims

| Place of search | Date of completion of the search | Examiner |
|---|---|---|
| The Hague | 7 November 2016 | Piret, Bernard |

Europäisches
Patentamt

European
Patent Office

Office européen
des brevets

# EUROPEAN SEARCH REPORT

Application Number

EP 15 38 2590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BURG C ET AL: "Leukaemia Inhibitory Factor derived from rat colon carcinoma cells increases host susceptibility to tumour growth", CYTOKINE, ACADEMIC PRESS LTD, PHILADELPHIA, PA, US, vol. 7, no. 8, 1 November 1995 (1995-11-01), pages 784-792, XP002980337, ISSN: 1043-4666, DOI: 10.1006/CYTO.1995.0094 * X for Invention 3; page 787 - page 788 * | 1-14 | |
| A | BRUNO ROSA ET AL: "Effectiveness and safety of monoclonal antibodies for metastatic colorectal cancer treatment: systematic review and meta-analysis", ECANCERMEDICALSCIENCE, vol. 9, 15 October 2015 (2015-10-15), XP55315738, ISSN: 1754-6605, DOI: 10.3332/ecancer.2015.582 | 1-14 | |
| X | HAIYANG YU ET AL: "LIF negatively regulates tumour-suppressor p53 through Stat3/ID1/MDM2 in colorectal cancers", NATURE COMMUNICATIONS, vol. 5, 17 October 2014 (2014-10-17), page 5218, XP055264997, DOI: 10.1038/ncomms6218 * X for Invention 3; page 8 - page 10; figure 2f * | 1-14 | TECHNICAL FIELDS SEARCHED (IPC) |

-/--

The present search report has been drawn up for all claims

| Place of search | Date of completion of the search | Examiner |
|---|---|---|
| The Hague | 7 November 2016 | Piret, Bernard |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 15 38 2590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GUIMBAUD ROSINE ET AL: "Leukemia inhibitory factor involvement in human ulcerative colitis and its potential role in malignant course", EUROPEAN CYTOKINE NETWORK, JOHN LIBBEY EUROTEXT LTD, FR , vol. 9, no. 4 1 December 1998 (1998-12-01), pages 607-612, XP008182109, ISSN: 1148-5493 Retrieved from the Internet: URL:http://www.jle.com/en/revues/ecn/e-doc s/leukemia_inhibitory_factor_involvement_i n_human_ulcerative_colitis_and_its_potenti al_role_in_malignant_course._90166/article .phtml * X for invention 3; the whole document * | 1-14 | |
| X | DANIELLE N. SETO ET AL: "A Key Role for Leukemia Inhibitory Factor in C26 Cancer Cachexia", JOURNAL OF BIOLOGICAL CHEMISTRY, vol. 290, no. 32, 7 August 2015 (2015-08-07), pages 19976-19986, XP055264989, US ISSN: 0021-9258, DOI: 10.1074/jbc.M115.638411 * X for invention 3; the whole document * | 1-14 | TECHNICAL FIELDS SEARCHED (IPC) |
| E | WO 2016/040657 A1 (UNIV CALIFORNIA [US]) 17 March 2016 (2016-03-17) * the whole document * | 1-15 | |

The present search report has been drawn up for all claims

| Place of search | Date of completion of the search | Examiner |
|---|---|---|
| The Hague | 7 November 2016 | Piret, Bernard |

Europäisches
Patentamt

European
Patent Office

Office européen
des brevets

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches
Patentamt

European
Patent Office

Office européen
des brevets

**LACK OF UNITY OF INVENTION
SHEET B**

Application Number

EP 15 38 2590

The Search Division considers that the present European patent application does not comply with the
requirements of unity of invention and relates to several inventions or groups of inventions, namely:

1. claims: 1-14(partially)

    (Use of) an agent that inhibits the expression or blocks the
    activity of leukemia inhibitory factor (LIF) to treat
    ovarian cancer

    ---

2. claims: 15(completely); 1-14(partially)

    (Use of) an agent that inhibits the expression or blocks the
    activity of leukemia inhibitory factor (LIF) to treat lung
    cancer

    ---

3. claims: 1-14(partially)

    (Use of) an agent that inhibits the expression or blocks the
    activity of leukemia inhibitory factor (LIF) to treat
    colorectal cancer

    ---

## ANNEX TO THE EUROPEAN SEARCH REPORT
## ON EUROPEAN PATENT APPLICATION NO.

EP 15 38 2590

This annex lists the patent family members relating to the patent documents cited in the above-mentioned European search report.
The members are as contained in the European Patent Office EDP file on
The European Patent Office is in no way liable for these particulars which are merely given for the purpose of information.

07-11-2016

| Patent document cited in search report | | Publication date | Patent family member(s) | | Publication date |
|---|---|---|---|---|---|
| WO 2015040243 | A2 | 26-03-2015 | EP | 3049110 A2 | 03-08-2016 |
| | | | US | 2016237158 A1 | 18-08-2016 |
| | | | WO | 2015040243 A2 | 26-03-2015 |
| US 2015133376 | A1 | 14-05-2015 | CN | 104623663 A | 20-05-2015 |
| | | | TW | 201517918 A | 16-05-2015 |
| | | | US | 2015133376 A1 | 14-05-2015 |
| WO 2011124566 | A1 | 13-10-2011 | AU | 2011237922 A1 | 01-11-2012 |
| | | | CA | 2797257 A1 | 13-10-2011 |
| | | | CN | 103797031 A | 14-05-2014 |
| | | | EA | 201201373 A1 | 30-04-2013 |
| | | | EP | 2371860 A1 | 05-10-2011 |
| | | | EP | 2556091 A1 | 13-02-2013 |
| | | | EP | 3045473 A1 | 20-07-2016 |
| | | | HK | 1208469 A1 | 04-03-2016 |
| | | | IL | 222270 A | 30-06-2016 |
| | | | JP | 2013523796 A | 17-06-2013 |
| | | | KR | 20130121679 A | 06-11-2013 |
| | | | SG | 184429 A1 | 29-11-2012 |
| | | | US | 2013142808 A1 | 06-06-2013 |
| | | | US | 2015139989 A1 | 21-05-2015 |
| | | | WO | 2011124566 A1 | 13-10-2011 |
| WO 2016040657 | A1 | 17-03-2016 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010115868 A
- US 6110462 A
- US 5093246 A
- US 5654157 A
- WO 2005030803 A
- WO 9601319 A

**Non-patent literature cited in the description**

- **KAMOHARA et al.** *Int J Oncol,* 2007, vol. 30, 977-983
- **CHENG et al.** *Biol Reprod,* 2004, vol. 1 70, 1270-1276
- **WHITE et al.** J.Biol.Chem. *Proc. Natl. Acad. Sci. USA,* 2007, vol. 104, 19357-19362
- **BELL et al.** *J. Rheumatol.,* 1997, vol. 24, 2394
- **KOHLER ; MILSTEIN et al.** *Nature,* 1975, vol. 256, 495
- **KIM et al.** *J. Immunol. Meth.,* 1992, vol. 156, 9-17
- **ALPHONSO et al.** Abstracts of the 28th National Meeting of the Society for Leukocyte Biology. *J. Leukocyte Biology,* 1991, vol. O, 49
- **HUDSON et al.** *J .Biol.Chem.,* 1996, vol. 271, 11971-11978
- **FAIRLIE/ W.D. et al.** *J.Biol.Chem.,* 2004, vol. 279, 2125-2134
- **METZ; S. et al.** *J.Biol.Chem.,* 2008, vol. 283, 5985-5995
- **SAMBROOK et al.** Molecular cloning: a Laboratory Manual. Cold Spring Harbor Laboratory Press, 2001, vol. 1-3